(19) **Europäisches Patentamt / European Patent Office / Office européen des brevets**

(11) **EP 4 517 775 B1**

(12) # EUROPEAN PATENT SPECIFICATION

(45) Date of publication and mention of the grant of the patent:
**15.04.2026 Bulletin 2026/16**

(21) Application number: **24172565.4**

(22) Date of filing: **25.04.2024**

(51) International Patent Classification (IPC):
**G16H 50/20** (2018.01)   **G16H 50/70** (2018.01)
**G16H 50/30** (2018.01)

(52) Cooperative Patent Classification (CPC):
**G16H 50/20; G16H 50/30; G16H 50/70**

(54) **SEPSIS DIAGNOSIS AND PREDICTION**

SEPSISDIAGNOSE UND -VORHERSAGE

DIAGNOSTIC ET PRÉDICTION DE LA SEPTICÉMIE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **28.08.2023 IN 202311057599**

(43) Date of publication of application:
**05.03.2025 Bulletin 2025/10**

(73) Proprietor: **FUJITSU LIMITED**
**Kawasaki-shi, Kanagawa 211-8588 (JP)**

(72) Inventors:
- **BAJPAI, Supriya**
  **560037 Bangalore (IN)**
- **DHARAMSHI, Nimish Mahendra**
  **560037 Bangalore (IN)**

(74) Representative: **Haseltine Lake Kempner LLP**
**Cheapside House**
**138 Cheapside**
**London EC2V 6BJ (GB)**

(56) References cited:
- **JOSEPH FUTOMA ET AL: "Learning to Detect Sepsis with a Multitask Gaussian Process RNN Classifier", ARXIV.ORG, CORNELL UNIVERSITY LIBRARY, 201 OLIN LIBRARY CORNELL UNIVERSITY ITHACA, NY 14853, 13 June 2017 (2017-06-13), XP080958782**
- **MARGHERITA ROSNATI ET AL: "MGP-AttTCN: An Interpretable Machine Learning Model for the Prediction of Sepsis", ARXIV.ORG, CORNELL UNIVERSITY LIBRARY, 201 OLIN LIBRARY CORNELL UNIVERSITY ITHACA, NY 14853, 11 May 2021 (2021-05-11), XP081948257, DOI: 10.1371/JOURNAL.PONE.0251248**
- **BAGHAEI KOUROSH T ET AL: "Sepsis Prediction: An Attention-Based Interpretable Approach", 2019 IEEE INTERNATIONAL CONFERENCE ON FUZZY SYSTEMS (FUZZ-IEEE), IEEE, 23 June 2019 (2019-06-23), pages 1 - 6, XP033626772, DOI: 10.1109/FUZZ-IEEE.2019.8858808**
- **MAX HORN ET AL: "Set Functions for Time Series", ARXIV.ORG, CORNELL UNIVERSITY LIBRARY, 201 OLIN LIBRARY CORNELL UNIVERSITY ITHACA, NY 14853, 14 September 2020 (2020-09-14), XP081761198**

## Description

FIELD OF INVENTION

The present invention relates to sepsis diagnosis/prediction, and in particular to a computer-implemented method, a computer program, and an information programming apparatus.

BACKGROUND OF INVENTION

Sepsis may be defined as a body's extreme response to an infection. It is a lifethreatening medical emergency. Sepsis occurs when an infection already present triggers a chain reaction throughout the body. Sepsis can lead to tissue damage, organ failure, and death.

Sepsis diagnosis/prediction is high in demand as an accurate prediction may save human lives and hospital resources. In particular, early sepsis diagnosis/prediction is desirable. Sepsis diagnosis/prediction may for example be carried out in an ICU (intensive care unit) setting.

In light of the above, a method for sepsis prediction is desired.

Reference may be made to a paper by Joseph Futoma et al titled "Learning to Detect Sepsis with a Multitask Gaussian Process RNN Classifier", arxiv.org, Cornell University Library, 201 Olin Library Cornell University Ithaca, NY 14853, 13 June 2017 (2017-06-13), XP080958782.

SUMMARY OF INVENTION

The present invention is defined by the independent claims, to which reference should now be made. Specific embodiments are defined in the dependent claims.

According to an embodiment of a first aspect there is disclosed herein a computer-implemented method as defined in claim 1.

The computer-implemented method may comprise performing the prediction process for the patient a plurality of instances, using input data covering a longer and/or more recent time period at each consecutive instance.

The computer-implemented method may comprise performing the prediction process each hour for a given number of hours.

The physiological measurement variables may comprise at least two of: heart rate; oxygen saturation; temperature; blood pressure (comprising any of systolic blood pressure, mean arterial pressure, and diastolic blood pressure); respiration rate; and end-tidal carbon dioxide.

The physiological measurement variables may comprise at least two of: heart rate; oxygen saturation; temperature; blood pressure (comprising any of systolic blood pressure, mean arterial pressure, and diastolic blood pressure); respiration rate; end-tidal carbon dioxide; blood sugar; Base Excess, bicarbonate, HCO3, level; fibrinogen level, platelets level; Fraction of Inspired Oxygen level pH level; Partial Pressure of Carbon Dioxide; oxygen saturation of arterial blood; Aspartate aminotransferase level; Blood urea nitrogen; Alkaline phosphatase level; Calcium level; Chloride level; Creatinine level; Direct Bilirubin level; Glucose level; Lactate level; Magnesium level; Phosphate level; Potassium level; Total Bilirubin level; Troponin I level; Haematocrit level; Haemoglobin level; Partial thromboplastin level; and White blood cell level.

The values of at least one of the physiological measurement variables may be obtained using at least one sensor (attached to the patient's body).

Generating the temporally updated embeddings may comprise using the RNN to compute/determine/find/recognize the second correlations between the first updated node embeddings.

The prediction process may comprise: generating multi-dimensional feature encodings based on the input data; and generating initial node embeddings by multiplying the multi-dimensional feature encodings with weight vectors, wherein the computing the first correlations comprises computing the first correlations based on the initial node embeddings.

Generating the multi-dimensional feature encodings may comprise, for each physiological measurement variable, performing a data binning method on the values concerned (and generating, as the multi-dimensional feature encodings, a feature vector for each value).

The prediction process may comprise generating an initial node embedding for each value of the input data, and wherein the computation of the first correlations comprises computing the first correlations based on the initial node embeddings.

Generating the initial node embeddings may comprise, for each physiological measurement variable, performing a data binning method on the values concerned to generate multi-dimensional feature encodings and multiplying the multi-dimensional feature encodings by weight vectors.

Computing the short range temporal correlations may comprise computing correlations between values of the same and different physiological measurement variables at two consecutive time steps.

Computing the short range temporal correlations for a value of a physiological measurement variable at a time step may comprise computing correlations between that value and the value of each physiological measurement variable at the previous time step.

The computing the short range temporal correlations may comprise computing correlations between a value at one of the consecutive time steps and the value of each physiological measurement variable at another of the consecutive time steps.

Computing the short range temporal correlations may comprise: computing a correlation between a value of a first one of the physiological measurement variables at a primary time step and a value of a second one of the physiological measurement variables at a secondary time step; computing a correlation between a value of the first one of the physiological measurement variables at the secondary time step and a value of the second one of the physiological measurement variables at the primary time step; computing a correlation between the value of the first one of the physiological measurement variables at the primary time step and the value of the first one of the physiological measurement variables at the secondary time step; and computing a correlation between the value of the second one of the physiological measurement variables at the primary time step and the value of the second one of the physiological measurement variables at the secondary time step, and the primary and secondary time steps may be consecutive time steps.

Computing the short range temporal correlations for a pair of consecutive time steps may comprise computing said correlations between values of the physiological measurement variables at the two consecutive time steps, and computing the first correlations may comprise computing said short range temporal correlations for each pair of consecutive time steps.

Computing the short range temporal correlations for a pair of consecutive primary and secondary time steps may comprise: computing a correlation between a value of a first one of the physiological measurement variables at the primary time step and a value of a second one of the physiological measurement variables at the secondary time step; computing a correlation between a value of the first one of the physiological measurement variables at the secondary time step and a value of the second one of the physiological measurement variables at the primary time step; computing a correlation between the value of the first one of the physiological measurement variables at the primary time step and the value of the first one of the physiological measurement variables at the secondary time step; and computing a correlation between the value of the second one of the physiological measurement variables at the primary time step and the value of the second one of the physiological measurement variables at the secondary time step, and computing the first correlations may comprise computing said short range temporal correlations for each pair of consecutive time steps.

Generating the first correlations may comprise using the initial node embeddings corresponding to the values, respectively.

Computing the short range temporal correlations for the values corresponding to the second time step in the order of time steps may comprise computing correlations between initial node embeddings corresponding to the second time step and initial node embeddings corresponding to the first time step (in the order of time steps).

Computing the short range temporal correlations for the values corresponding to the second time step in the order of time steps may comprise computing correlations between each of the initial node embeddings corresponding to the second time step and each of the initial node embeddings corresponding to the first time step (in the order of time steps).

Computing the short range temporal correlations for the values corresponding to the second time step in the order of time steps may comprise: computing a correlation between an initial node embedding corresponding to a first one of the physiological measurement variables and corresponding to the/a first time step (preceding the second time step) and an initial node embedding corresponding to a second one of the physiological measurement variables and corresponding to the second time step; and computing a correlation between an initial node embedding corresponding to the first one of the physiological measurement variables and corresponding to the second time step and an initial node embedding corresponding to the second one of the physiological measurement variables and corresponding to the first time step; computing a correlation between the initial node embedding corresponding to the first one of the physiological measurement variables and corresponding to the first time step and the initial node embedding corresponding to the first one of the physiological measurement variables and corresponding to the second time step; and computing a correlation between the initial node embedding corresponding to the second one of the physiological measurement variables and corresponding to the first time step and the initial node embedding corresponding to the second one of the physiological cal measurement variables and corresponding to the second time step.

Computing the short range temporal correlations for the values corresponding to each of the third and subsequent time steps in the order of time steps may comprise computing correlations between each of the initial node embeddings corresponding to the time step concerned and each of the first updated node embeddings corresponding to the preceding time step.

Computing the short range temporal correlations for the values corresponding to the second time step in the order of time steps may comprise computing correlations between each of the initial node embeddings corresponding to the second time step and each of the initial node embeddings corresponding to the first time step (in the order of time steps), and computing the short range temporal correlations for the values corresponding to each of the third and subsequent time steps in the order of time steps may comprise computing correlations between each of the initial node embeddings corresponding to the time step concerned and each of the first updated node embeddings corresponding to the preceding time step.

The prediction process may comprise updating the initial node embeddings based on the short range temporal correlations to generate intermediate node embeddings.

The prediction process may comprise updating each initial node embedding with/based on its short range temporal correlations with the (initial or updated) node embeddings of the preceding time step to generate intermediate node embeddings.

Updating each initial node embedding with/-based on its short range temporal correlations to generate intermediate node embeddings may comprise using a weighted sum.

Updating an initial node embedding to generate an intermediate node embedding may comprise adding a contribution to the intermediate node embedding based on each (initial or updated) node embedding of the previous time step weighted by its (short range temporal) correlation with the initial node embedding concerned.

Generating the spatial correlations may comprise computing correlations between the intermediate node embeddings corresponding to a same time step (and corresponding to different physiological measurement variables).

Generating the first updated node embeddings may comprise updating each intermediate node embedding with/based on its (spatial) correlation with each other intermediate node embedding corresponding to the same time step.

Computing the short range temporal correlations for the values corresponding to the second time step in the order of time steps may comprise computing correlations between each of the initial node embeddings corresponding to the second time step and each of the initial node embeddings corresponding to the first time step (in the order of time steps); and computing the short range temporal correlations for the values corresponding to each of the third and subsequent time steps in the order of time steps may comprise computing correlations between each of the initial node embeddings corresponding to the time step concerned and each of the first updated node embeddings corresponding to the preceding time step.

The computing the short range temporal correlations may comprise computing the short range temporal correlations for the values corresponding to a second time step and for the values corresponding to a third time step and for the values corresponding to subsequent time steps, the second time step occurring after a first time step and before the third time step; the computing the short range temporal correlations for the values corresponding to the second time step may comprise computing correlations between each of the initial node embeddings corresponding to the second time step and each of the initial node embeddings corresponding to the first time step; and the computing the short range temporal correlations for the values corresponding to each of the third and subsequent time steps may comprise computing correlations between each of the initial node embeddings corresponding to the time step concerned and each of the first updated node embeddings corresponding to the said time step which precedes the time step concerned.

The generating the first updated node embeddings may comprise updating each intermediate node embedding with/based on its (spatial) correlation with each other intermediate node embedding corresponding to the same time step.

Updating each intermediate node embedding to generate the first updated node embeddings may comprise using a weighted sum.

Updating an intermediate node embedding to generate a first updated node embedding may comprise adding a contribution to the first updated node embedding based on each other intermediate node embedding of the same time step weighted by its (spatial) correlation with the intermediate node embedding concerned.

Generating the spatial and short range temporal correlations may comprise computing dot-products between the node embeddings concerned.

Generating the spatial correlations may comprise using a self-attention mechanism/network.

Generating the short range temporal correlations may comprise using an attention-based mechanism/network.

Generating the spatial correlations may comprise using a key-query-value self-attention mechanism/-network.

Generating the short range temporal correlations may comprise using a key-query-value attention-based mechanism/network.

The prediction process may comprise repeatedly generating said first correlations and updating the node embeddings concerned, comprising, for each subsequent iteration, starting with the first updated node embeddings of the previous iteration in place of the initial node embeddings.

The RNN may be configured (may have been trained) to learn long term temporal correlations between the first updated embedding vectors.

The RNN may comprise at least on gated recurrent unit, GRU.

The NN may have been trained to output a sepsis prediction.

The NN may be configured (may have been trained) to generate the prediction in the form of a binary classification task.

The NN may comprise at least one or a plurality of (fully-connected) layers.

The computer-implemented method may comprise performing a training process, the training process comprising: performing the prediction process using training data corresponding to a training patient as the input data; adjusting at least one (or every) network weight used in the attention-based mechanism, the self-attention mechanism, the RNN, and the NN based on a difference between the generated prediction/diagnosis and a training prediction/diagnosis corresponding to the training data (as a ground truth prediction/diagnosis/output) (to bring the generated prediction to or towards the training prediction).

The computer-implemented method may comprise performing the training process for a plurality of iterations using different training data for each iteration.

The computer-implemented method may comprise performing/iterating the training process until (an iteration in which) the difference between the generated prediction and the training prediction concerned converges or is below an error threshold.

The computer-implemented method may comprise performing/iterating the training process until a predefined number of successive iterations in which the difference between the generated prediction and the training prediction concerned is below an error threshold.

The computer-implemented method may comprise performing/iterating the training process a predefined number of iterations.

The computer-implemented method may comprise, after performing the training process, performing the prediction process using target input data of a target patient to generate a target prediction/diagnosis.

The computer-implemented method may comprise performing the prediction process for the target patient a plurality of instances, using target input data covering a longer (and more recent) time period at each consecutive instance.

The computer-implemented method may comprise performing the prediction process each hour for a given number of hours.

According to an embodiment of a second aspect there is disclosed herein a computer program as defined in claim 13.

According to an embodiment of a third aspect there is disclosed herein an information processing apparatus as defined in claim 14.

Features relating to any aspect/embodiment may be applied to any other aspect/embodiment.

BRIEF DESCRIPTION OF DRAWINGS

Reference will now be made, by way of example, to the accompanying drawings, in which:

Figure 1 is a diagram illustrating a first comparative method;
Figure 2 is a diagram illustrating a first comparative method;
Figure 3 is a diagram illustrating a second comparative method;
Figure 4 is a diagram illustrating a prediction process;
Figure 5 is a diagram illustrating a prediction process;
Figure 6 is a diagram illustrating a prediction process;
Figure 7 is a diagram illustrating a prediction process;
Figure 8 is a diagram illustrating a prediction process;
Figure 9 is a table;
Figure 10 is a diagram illustrating a prediction process;
Figure 11 is a diagram illustrating a training process and a test process;
Figure 12 is a diagram illustrating a prediction process; and
Figure 13 is a diagram illustrating an apparatus.

DETAILED DESCRIPTION

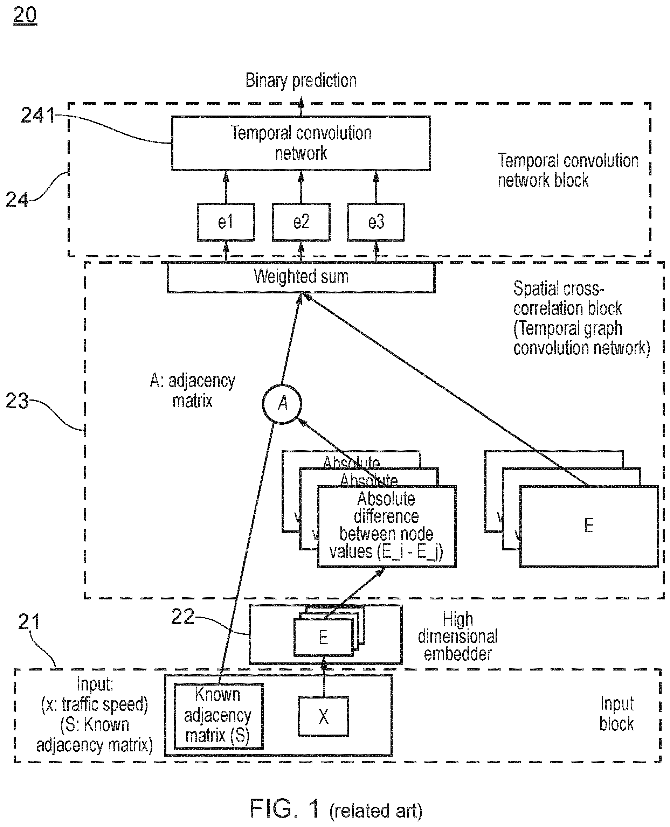

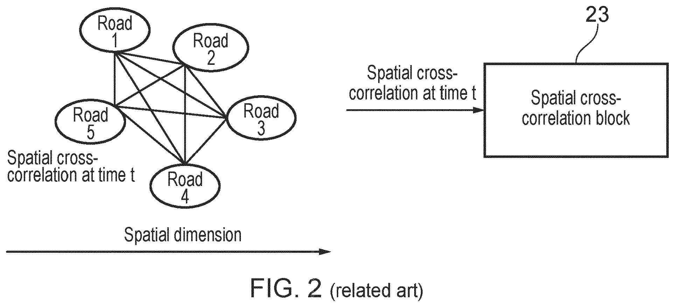

Figures 1 and 2 are diagrams illustrating a first comparative method (comparative method 1). Comparative method 1 is a method for road network traffic forecasting. Such traffic forecasting involves the use of spatial and temporal dependencies in the traffic network. It is described below why such a method is not suitable or useful for sepsis prediction based on physiological parameters. A known adjacency matrix A is used which is based on road networks. The adjacency matrix A reduces the learning space of a spatial cross-correlation block 23 (which may be referred to herein as a spatial features block). As shown in the input block 21 input data is time series data of traffic speed (x). A high dimensional embedder 22 generates a high dimensional representation (E) of the input data x.

The spatial features block 23 dynamically computes edge weights based on the static adjacency matrix of road networks and the absolute difference between the high dimensional representation (maybe referred to as node values) E. The vectors "e" are generated based on the edge weights and the input data (i.e. using a weighted sum). Figure 2 illustrates the learning of the spatial correlations (may be referred to as cross-correlations) between data of different roads in the so-called spatial dimension (not across time).

In a temporal convolution block 24 (specifically a temporal convolution network 241), a dilated convolution takes place on data frame vectors (e) of different time stamps (i.e. $e_1$, $e_2$, etc.) to obtain a time-based aggregation of data. Based on the output of the temporal convolution network 241, a binary prediction is made.

The methodology of comparative method 1 is not suitable or useful for sepsis prediction based on physiological parameters. This is because, for example, unlike the input data in comparative method 1 (traffic speed), physiological parameters data of different physiological variables do not share similar attributes (e.g. HR (heart rate) and O2Sat (O2 saturation) do not share similar attributes - at least, not sufficiently for the methodology of comparative method 1 to be useful), hence the absolute difference between them does not hold any useful meaning in capturing edge strength (weight).

Furthermore, in comparative method 1 the initial edges (weights) are known (i.e. via the static adjacency matrix of road networks). The edges are merely updated at each time step using the previous traffic speed data using a graph convolution network. There is no such known adjacency matrix available in the case of sepsis prediction based on physiological parameters data.

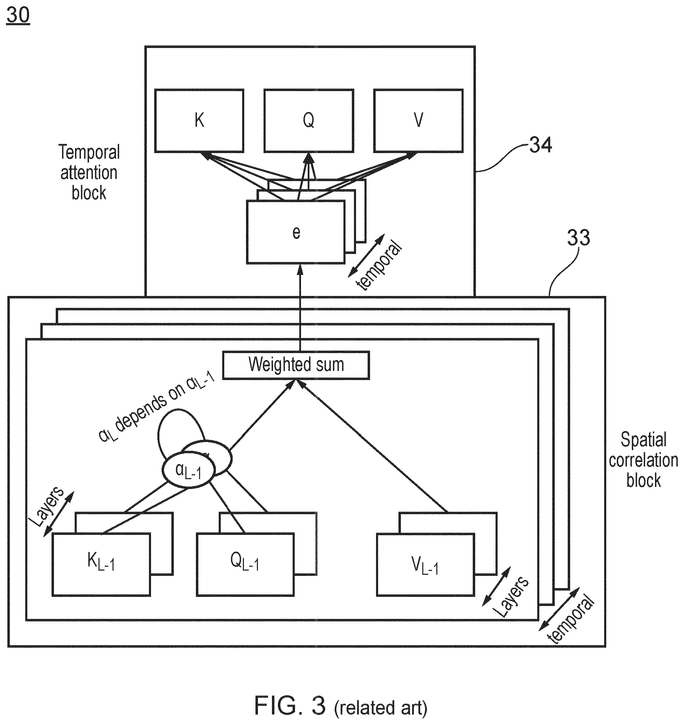

Figure 3 is a diagram illustrating a second comparative method (comparative method 2). Comparative method 2 is for dealing with irregularly sampled multivariate time series data. In a spatial correlation block 33 (may be referred to as a spatial feature extraction block) illustrated in Figure 3, each layer gets computed node embeddings and edge weights of the previous layer as input and computes current node embeddings as the output, which is used to dynamically compute the edge weights of the current layer. This computation multiplies the previous layer edge weights with a temporal aggregation of attention between node embeddings (temporal aggregation means from average of several given time series samples). Therefore a connection which ceases to exist in lower layers may well vanish in upper layers as well. Each layer in this comparative method represents a different variable. For the edge weights computation in the spatial correlation block, a temporal based aggregation of attention between node embeddings is done, which is sensitive to the order of time of a given dataframe. The vectors e are generated based on the last layer edge weights and the node embeddings (i.e. using a weighted sum). In a temporal attention block 34 as illustrated, temporal self-attention is used. In this block a step is performed of concatenating the embedding of time step to key, query and value.

A problem with applying the methodology of comparative method 2 to a sepsis prediction based on time series physiological parameters data is that useful temporal relationships between different variables are not captured by the spatial correlation generation nor by the time-aggregation of the attention weights between nodes. Another problem is that edge weights should not be computed based on previous layer edge weights because each layer represents different information corresponding to different physiological parameters.

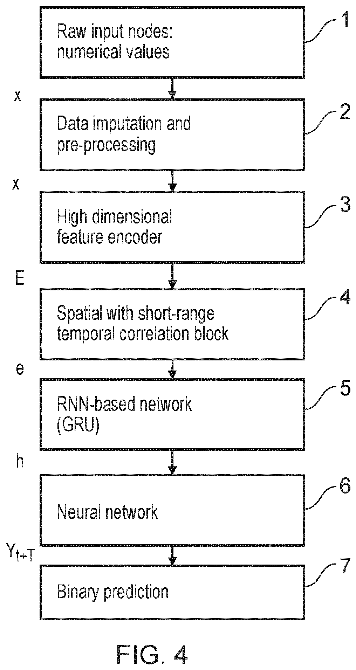

Figure 4 is a diagram illustrating a prediction process.

At step 1, (raw) data (indicated by "x" in Figure 4) is input. The data comprises physiological data of a patient in the form of continuous floating point numerical values recorded at regular or irregular time intervals, for example hourly. For example, the data is recorded in an ICU setting and/or using at least one sensor attached to the patient's body. Additional data in the form of lab report data (continuous floating point numerical values) recorded infrequently may also be used. The physiological data may be referred to as values of physiological measurement variables of/related to the patient over a time period. The input data comprises multi-variate time series data. A single feature at a single time step is a scalar value. Features and physiological measurement variables may be used interchangeably. Features/physiological measurement variables may comprise vitals data, e.g. vital signs data (body temperature, pulse rate, respiration rate, blood pressure, blood oxygen saturation etc), which are recorded frequently as well as cardiovascular data, respiration data, metabolic data, and immunological data which are recorded infrequently.

At step 2, data imputation and preprocessing is performed. For example, data imputation is a method for retaining the majority of a dataset's data and information by substituting missing data with a different value. Data preprocessing comprises, for example, cleaning, transforming, and integrating of data in order to make it ready for analysis. The imputation and preprocessing will of course depend on the specific data which is input. The output of step 2 (the preprocessed data) is indicated by "x" in Figure 4 (as is the raw input data).

At step 3, a high dimensional feature representation of the input numerical features/vitals (Physiological parameters) is generated. The high dimensional feature representations may be referred to as high dimensional encodings or multi-dimensional encodings and are indicated as "E" in Figure 4. In other words, each scalar value is mapped to a high dimensional space for better expressive power.

At step 4 the processing of a correlation block (or "spatial with short-range temporal correlation block") takes place. Here, first correlations are generated between the values of the physiological measurement variables as described later. In short, the spatial pattern (cross-correlation between features at the same time step, e.g. time t) is captured by this block, as well as the cross-correlation between features of the time t and t-1 (i.e. short-range temporal correlations across two consecutive time steps). As described later, the calculation of correlations in such a way extracts the spatial as well as short term temporal information which is more informative. This block generates first updated node embeddings ("e") based on the high dimensional encodings and the first correlations.

At step 5, a recurrent neural network (RNN), or an RNN-based network, comprising at least one gated recurrent unit (GRU) generates long-term temporal patterns of the features, i.e. second correlations between the first updated node embeddings. As described later, the long-term temporal patterns of the features are captured using the GRU which improves the sepsis prediction. The RNN generates temporally updated embeddings ("h") based on the first updated node embeddings and the second correlations.

At step 6, a neural network (NN) is used to generate a prediction of sepsis or no sepsis (i.e. as a binary classification task), e.g. at time t+T. The NN comprises fully connected layers. The binary prediction is indicated by "$Y_{t+T}$" in Figure 4.

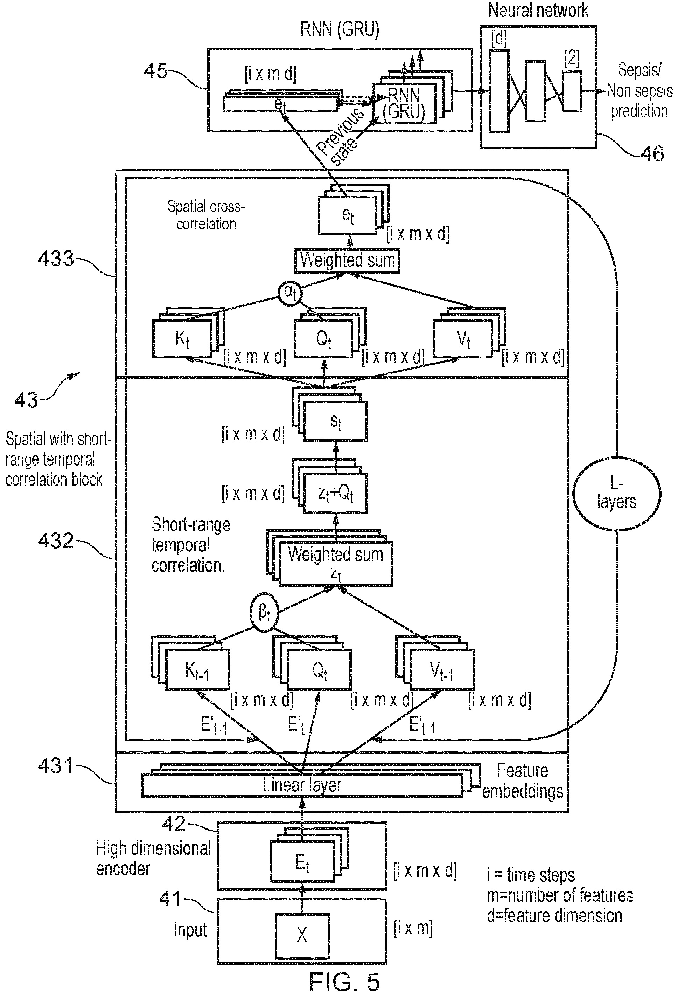

Figure 5 is a diagram illustrating a representation of a prediction process using example architecture according to an implementation and may be considered a more detailed representation of the Figure 4 prediction process. The architecture comprises an input 41, a high dimensional encoder 42, a spatial with short range temporal correlation block 43 (which may be referred to as a correlation block and which comprises a feature embeddings block 431, a short range temporal correlation block 432, and a spatial cross-correlation block 433), a recurrent neural network (RNN) 45, and a neural network (NN) 46.

As illustrated, the input data has dimension i x m, where i is the number of time steps and m is the number of features. The high dimensional encoder 42 generates the high dimensional encodings or "high dimensional features" of the input data. The high dimensional encodings are represented as "E" in Figure 5 and have dimension i x m x d, where d is the feature dimension.

The encodings are input to the correlation block 43 (as the input data) so that first correlations are computed. Computing the first correlations comprises computing short range temporal correlations between values of different physiological measurement variables at different time steps and between values of the same physiological measurement variable at different time steps using an attention-based mechanism (i.e. a modified attention mechanism) and computing spatial correlations between values of different physiological measurement variables at a same time step using a self-attention mechanism.

The correlation block 43 uses a key-query-value (KQV) scheme/mechanism to generate the short range (or short-term) temporal correlations (in the short range temporal correlation block 432) and the spatial correlations (in the spatial cross-correlation block 433). First, the encodings are multiplied with weight vectors (W, not shown) and a non-linear transformation is performed to generate initial node embeddings E' (as illustrated by the linear layers in the feature embeddings block 431). A different weight vector W is used for each feature in Figure 5, as described later. In the KQV scheme these initial node embeddings E' are further multiplied with further weight vectors to generate query, key, and value vectors (different weight vectors for key, query and value are used). The values of the features/variables, or the encodings, or the embeddings, may be referred to as nodes. That is, "node" may be considered to correspond to a particular feature/physiological measurement variable, in the sense that an initial node embedding corresponds to a value of a physiological measurement variable at a time step. For example, temperature is a node and e.g. blood pressure is another node, and e.g. respiration rate is a third node.

The first correlations of a given node (i.e. a given variable at time t) are determined in two stages. In the first stage (in block 432), short range temporal correlations are determined and in the second stage (in block 433) spatial correlations are determined.

To generate the short-term temporal correlations, data (encodings) of consecutive pairs of time steps are considered. Considering, for example, the consecutive times steps t=0 and t=1, for the key and value (vectors K, V) data of time step t=1 is used, while for the query (vector Q) data of time step t=0 is used, and correlation weights (beta), which may be referred to as short range temporal correlation weights, are learned. Specifically, a modified attention mechanism is used to generate the short range temporal correlations. To generate the spatial correlations, data (intermediate node embeddings s) of each time step are considered separately, and the correlation weights (alpha), which may be referred to as spatial correlation weights, are determined using the key-query-value mechanism. Specifically, a self-attention mechanism is used to generate the spatial correlations. The output of the correlation block 43 is first updated node embeddings "e".

In the first stage, intermediate node embeddings ("s") are generated, which may be referred to as short range temporal embeddings. This is done by determining the correlations between nodes of time t-1 and time t using a modified attention mechanism (as described later in detail), which are referred to as short range temporal correlations, and updating the initial node embeddings with the correlations to generate the intermediate node embeddings (as in Figure 5). As described later, when generating the intermediate node embeddings for the second time step, the initial node embeddings are used as the node embeddings of the previous time step to compute the correlations. When generating the intermediate node embeddings for time steps for which first updated node embeddings of the previous time step exist (i.e. for the third and subsequent time steps), those first updated node embeddings of the previous time step are used in place of the initial node embeddings as the node embeddings of the previous time step to compute the correlations. This is illustrated in Figure 5 by the arrow on the left extending from the first updated node embeddings to the initial node embeddings. That is, $e_{t-1}$ is used in place of $E'_{t-1}$. Thus, the first updated node embeddings are computed for each time step in turn (e.g. the first updated node embeddings for time step t=4 is computed before the first updated node embeddings for the time step t=5).

In the second stage, the intermediate node embeddings generated in the first stage are used as input to determine the first updated node embeddings (may be referred to as spatial node embeddings) of each node (i.e. for each feature) using a self attention mechanism (described later in detail). That is, spatial correlations are determined between the intermediate node embeddings of the same time step and used to update the intermediate node embeddings to generate the first updated embeddings (as shown in Figure 5).

Effectively, the initial node embeddings E' are updated with the first correlations (spatial and short range temporal) to generate first updated node embeddings e.

As illustrated, a weighted sum is used in the computation of the intermediate node embeddings and the first updated node embeddings (i.e. an initial node embedding at time t=1 is updated based on node embeddings at time t=0 weighted by the relevant short range temporal correlation weight (beta), and an intermediate node embedding at time t=1 is updated based on the other intermediate node embeddings at time t=1 weighted by the relevant spatial correlation weight (alpha)).

The first correlation computation and update processing is carried out L times, where L is a positive integer of one or more. That is, the processing may be repeated. This is indicated by the "L-layers" text in Figure 5. The correlation block 43 may be considered a single layer of the overall correlation block, in the sense that each layer carries out an iteration of the first correlation computation and update processing. Repeated iterations use the first updated node embeddings of the previous iteration (layer) in place of the initial node embeddings.

The initial, intermediate, and first updated node embeddings may be referred to as initial, intermediate, and first updated: feature embeddings; embeddings; embedding vectors; or feature embedding vectors.

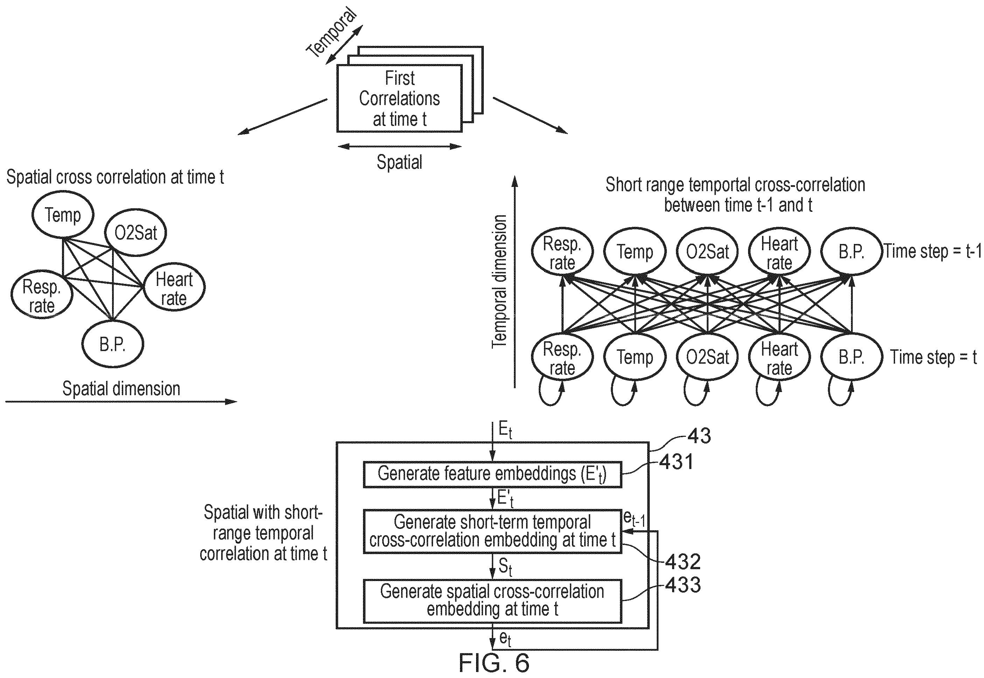

Figure 6 is useful for understanding the difference between what are referred to as the spatial correlations and the short range temporal correlations. On the left are represented the spatial correlations which are between variables at the same time step (e.g. t). On the right are represented the short range temporal correlations which are between pairs of variables at two consecutive time steps e.g. t and t-1 (including between the same variable at the two consecutive time steps). These, together, are the first correlations. As illustrated at the top of Figure 6, the first correlations comprise a set of first correlations for each time step. Any of the correlations may be referred to as cross-correlations herein. The first correlation computation (and the difference between the spatial correlation computation and the short range temporal correlation computation) is described in more detail later.

At the bottom of Figure 6 is shown the overall correlation computation and update processing to generate the intermediate node embeddings (or "short-term temporal cross-correlation embeddings") at time t, st, and the first updated node embeddings (or "spatial cross-correlation embedding") at time t, et, and the way that for the relevant time steps the first updated node embeddings of the previous time step, $e_{t-1}$, is used in the computation of the intermediate node embeddings, $s_t$. The overall correlation computation and update processing is illustrated as the correlation block 43 including the blocks 431, 432, and 433 described above.

Returning to Figure 5, the initial, intermediate, and first updated node embeddings E', s, and e have the same dimensions.

These first updated node embeddings are concatenated and input to the RNN 45. This step may be considered inputting the first updated node embeddings into the RNN 45.

The RNN 45 processes the first updated node embeddings to learn long-range temporal correlations between them and updates the input with these correlations, which may be referred to as second correlations. The outputs of the RNN 45 are referred to as temporally updated embeddings.

The temporally updated embeddings are input to the NN 46. The temporally updated embeddings include information of the first correlations and the second correlations and thus information about patterns across the different features and different time steps. The NN 46 has been trained to generate a sepsis/non-sepsis prediction based on such data as a binary classification task. The NN 46 makes the prediction.

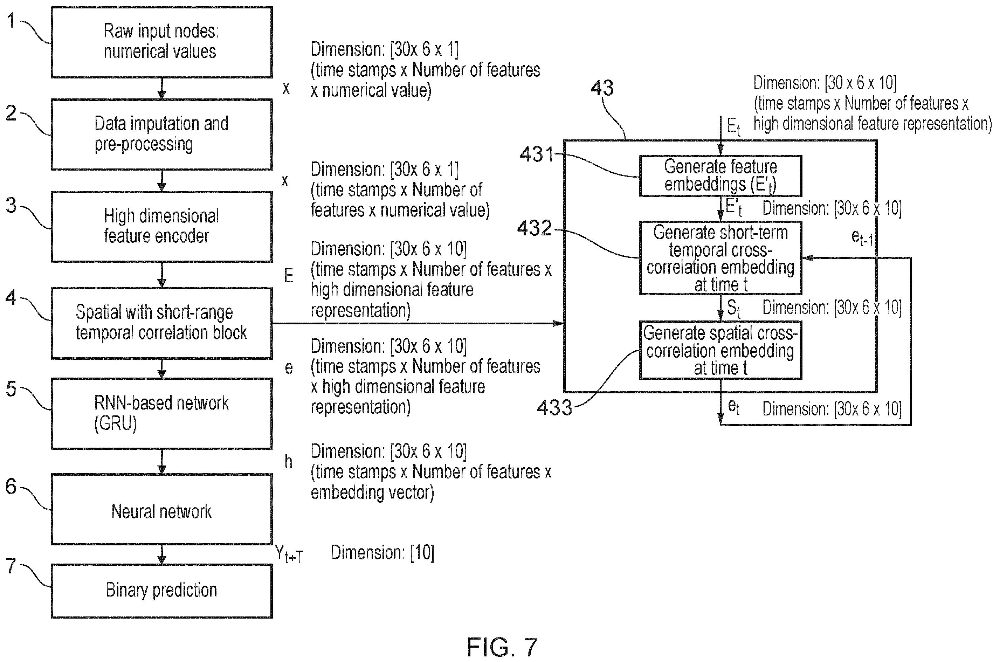

Figure 7 is a diagram illustrating the prediction process of Figure 4 and includes some example dimensions for aid in understanding the process. In this example, the input data x has dimension 30 x 6 x 1; the high-/multi dimensional features/encodings E have dimension 30 x 6 x 10 (that is, each feature has encoding vector dimension of 10, i.e. 10 x 1); the first updated node embeddings e (and the initial and intermediate node embeddings E' and s) have dimension 30 x 6 x 128 (that is, each feature has embedding vector dimension of 128, i.e. 128 x 1); and the temporally updated embeddings h have dimension 128 which is equal to the number of hidden units in the GRU layer. The the overall correlation computation and update processing is illustrated on the right-hand side as the correlation block 43 as in Figure 6, and the arrow from step 4 indicates that this processing is performed in step 4.

A specific implementation of the first correlation computation will be described.

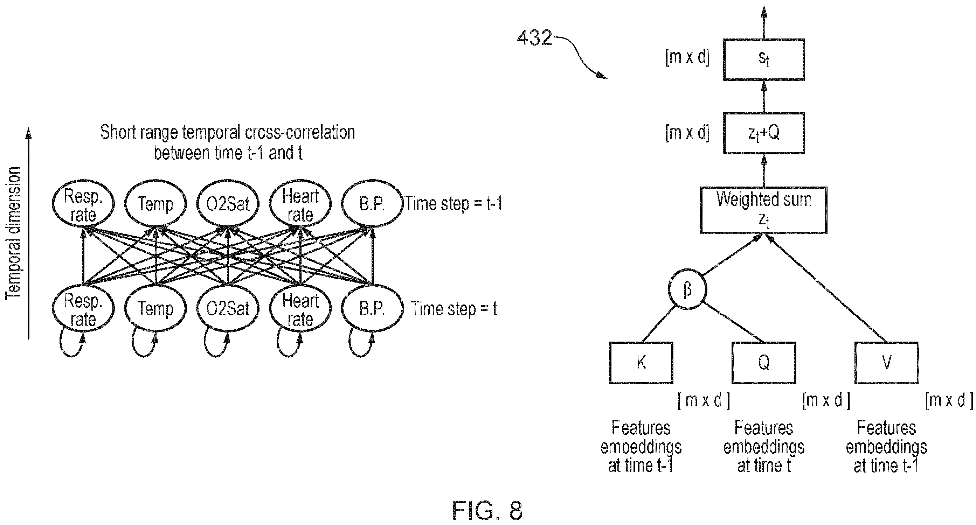

Figure 8 is useful for understanding the short range temporal correlation computation. In the computation of the short range temporal correlations, the key-query-value scheme is implemented using the following equations. The processing on the right-hand side is given the reference sign 432 indicating that it is the processing that occurs in the short range temporal correlation block 432.

"Key" vectors/matrices: $K = W_K e_{t-1}$, where $e_{t-1}$ represents the key features at time step t-1 (i.e. the embeddings "e" at t-1, that is, the first updated node embeddings at time t-1), and $W_K$ represents a weight vector for performing the linear transformation (multiplying embedding with weights).

"Query" vectors/matrices: $Q = W_Q E'_t$, where E't represents the query features at time step t (i.e. the embeddings "E'" at t, that is, the initial node embeddings at time t), and $W_Q$ represents a weight vector for performing the linear transformation.

"Value" vectors/matrices: $V = W_V e_{t-1}$, where $e_{t-1}$ represents the value features at time step t-1 (i.e. the embeddings "e" at t-1, that is, the first updated node embeddings at time t-1), and $W_V$ represents a weight vector for performing the linear transformation.

The weights $W_K$, $W_Q$, and $W_V$ for the key, query, and value are different from each other (not necessarily the same).

The attention weights $\beta$ represent the short-range temporal correlation weights between keys and queries, which are learned/trainable.

$\beta$ = softmax(Q.K$^T$ / sqrt(d)), where d is the dimension of query and key vector. The correlations use the dot product between Q and K.

Final short-range temporal embeddings (intermediate node embeddings) st of nodes are calculated as: st = zt + Q, where, $z_t = \beta$V is the weighted sum of the values V, and Q is the query vector.

These intermediate node embeddings s include information regarding the correlation of the physiological measurement variables (nodes) at time t with physiological measurement variables (nodes) at time t-1. They may be considered to also the includes the information of self-correlation (that is, due to the "+ Q" term), which for example may be considered useful as the intermediate node embeddings are used to compute the spatial correlations. This "self-correlation" is illustrated in Figures 6 and 8 by the arrows extending from each feature at time t back to itself. As previously mentioned, the processing may be referred to as attention.

Referring back to Figure 5, in the computation of the spatial correlations, the key-query-value scheme is implemented using the following equations.

"Key" vectors/matrices: K = $W_K$ $s_t$, where st represents the short-range temporal node embeddings (intermediate node embeddings) at time step t, and $W_K$ represents a weight for the linear layers of the key vector.

"Query" vectors/matrices: Q = $W_Q$ st, where st represents the short-range temporal node embeddings (intermediate node embeddings) at time step t, and $W_Q$ represents a weight for the linear layers of the query vector.

"Value" vectors/matrices: V = $W_V$ st, where st represents the short-range temporal node embeddings (intermediate node embeddings) at time step t, and $W_V$ represents a weight for the linear layers of the value vector.

The weights $W_K$, $W_Q$, and $W_V$ for the key, query, and value are different from each other (not necessarily the same), and also different (not necessarily the same) from the weights $W_K$, $W_Q$, and $W_V$ of the short-range temporal correlation computation.

For computing the spatial correlation at time t, a self-attention mechanism is used, and the correlation weights between physiological parameters at time t (nodes at time t) are learned. These weights represent spatial correlations (because they are computed among the same time step) and are denoted by $\alpha$ and defined by the equation:

$\alpha$ = softmax(Q.K$^T$ / sqrt(d)), where d is the dimension of query and key vector. The correlation weights $\alpha$ are calculated by performing the dot product between Q and K.

First updated node embeddings at time t (et) are computed by performing the weighted sum of values V, that is, et = $\alpha$V. These node embeddings (et) include information regarding the correlations between the physiological measurement variables (nodes) at same time step t. They also include the information of the short-range correlations of nodes between time step t and previous time step t-1.

As previously described, the processing above is repeated (with, for a given time step t, the first updated node embedding et of the previous iteration being used in place of the initial node embeddings E't of the following iteration, as indicated in the equations above). That is, the modified attention processing followed by the self-attention processing is repeated. This processing is performed for each time step. When considering the first time step there is no previous time step. In some implementations, the earliest data processed using the modified attention and self-attention processing is the data of the second time step so that the updated node embeddings for every time step include both spatial and short range temporal correlation information.

Returning to the processing of the short range temporal correlations (e.g. Figure 8), the following equations may be useful to aid understanding and may be considered a simplified partial explanation. Here, T, B, and R represent different physiological measurement variables. The suffix "1" indicates the value of that variable at a first time step (e.g. t=1) and the suffix "2" indicates the value of that variable at a following second time step (e.g. t=2).

The key (K), query (Q), and value (V) vectors may be represented as follows:

$$V = [T1, B1, R1]_{3 \times 128}$$

$$K = [T1, B1, R1]_{3 \times 128}$$

$$Q = [T2, B2, R2]_{3 \times 128}$$

The intermediate node embeddings may be represented as follows:

$$[(QK^T)_{3 \times 3} V_{3 \times 128}]_{3 \times 128} + Q_{3 \times 128}$$

The above equation can be expanded as follows:

$$T_2 = \beta_{11} T_1 + \beta_{12} B_1 + \beta_{13} R_1 + T_2,$$

where the weights $\beta 11$, $\beta 12$, $\beta 13$ are the attention/correlation weights.

$$B_2 = \beta_{21} T_1 + \beta_{22} B_1 + \beta_{23} R_1 + B_2 :$$

where the weights $\beta_{21}$, $\beta_{22}$, $\beta_{23}$ are the attention/correlation weights.

$$R2= \beta_{31}T_1+ \beta_{32}B_1+ \beta_{33}R_1+R_2 \quad :$$

where the weights $\beta_{31}$, $\beta_{32}$, $\beta_{33}$ are the attention/correlation weights.

The generation of the first updated node embeddings may be similarly represented, and in such a case the time step for all quantities would stay the same (e.g. t=2) and there is no "+Q" term.

It will be appreciated that the correlation computations described above may be considered to comprise computing correlations between the values represented by the node embeddings.

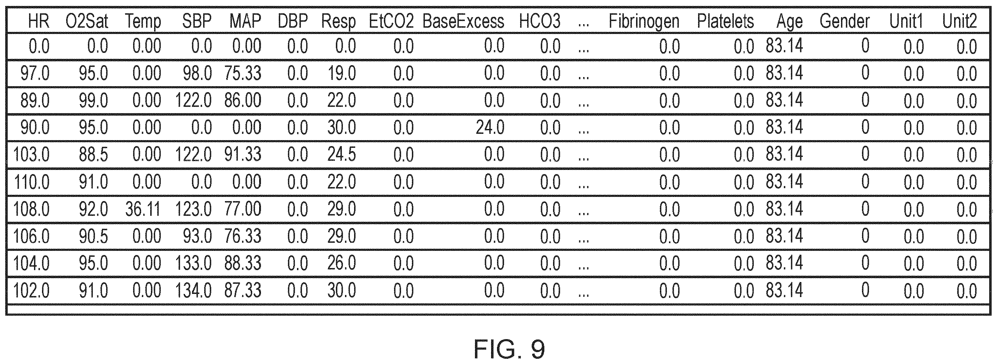

Figure 9 is a table illustrating some example input data. Here, the physiological measurement variables comprise: body temperature (Temp), heart rate (HR), respiration rate (Resp), blood oxygen saturation (O2Sat), systolic blood pressure (SBP), diastolic blood pressure (DBP), mean arterial pressure (MAP), end-tidal carbon dioxide (EtCO2).

The input data in Figure 9 includes the demographics data of 'Age', 'Gender'. The input data in Figure 9 includes as further physiological measurement variables the results of the laboratory/blood tests: Base Excess (BEs), Bicarbonate (HCO3), Fibrinogen, Platelets. Although not shown in Figure 9, input data may comprise (additionally or alternatively) as physiological measurement variables the results of any of the blood tests: Fraction of Inspired Oxygen (FiO2), pH, Partial Pressure of Carbon Dioxide (PaCO2), oxygen saturation of arterial blood (SaO2), Aspartate aminotransferase (AST), Blood urea nitrogen (BUN), Alkaline phosphatase (ALP), Calcium, Chloride, Creatinine, Direct Bilirubin, Glucose, Lactate, Magnesium, Phosphate, Potassium, Total Bilirubin, Troponin I, Haematocrit (Hct), Haemoglobin (Hgb), Partial thromboplastin (PTT), White blood cell (WBC).

Input data may comprise (additionally or alternatively) biomarkers (molecules) that are released into the blood or other body fluids in response to infection or inflammation such as procalcitonin (PCT), C-reactive protein (CRP), and interleukin-6 (IL-6).

In general, input data may comprise values of any of the above-mentioned physiological measurement variables.

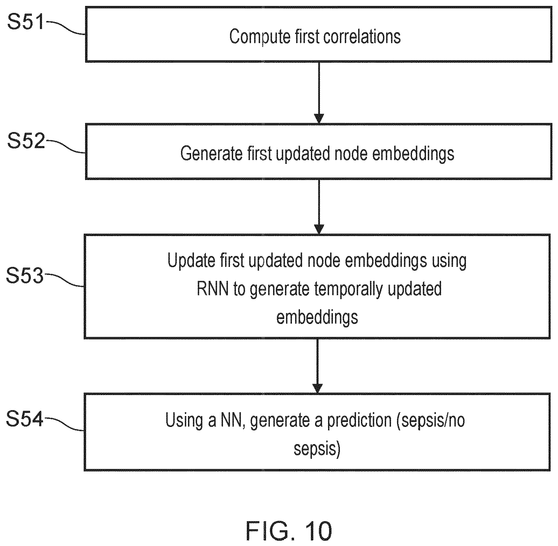

Figure 10 is a diagram illustrating a prediction process according to an implementation. The prediction process comprises steps S51-S54.

Step S51 comprises: based on input data comprising values of physiological measurement variables of a patient over a time period, computing first correlations in the input data, comprising computing short range temporal correlations between values of different physiological measurement variables at different time steps and between values of the same physiological measurement variable at different time steps using an attention-based mechanism and computing spatial correlations between values of different physiological measurement variables at a same time step using a self-attention mechanism.

Step S52 comprises generating first updated node embeddings based on the input data and the first correlations.

Step S53 comprises: using a recurrent neural network, RNN, updating the first updated node embeddings based on second correlations between the embedding vectors to generate temporally updated embeddings.

Step S54 comprises: based on the temporally updated embeddings and using a neural network, NN, generating a prediction/diagnosis indicating whether or not the patient has (or will have, i.e. at predetermined time in the future) sepsis.

Steps S51 and S52 are based on the high dimensional encodings/features as the input data. The prediction process may comprise generating the high dimensional encodings/features, for example: for each physiological measurement variable, performing a data binning method on the values concerned and generating, as the high dimensional encodings/features, a feature vector for each value.

Any of the steps S51-S54 may comprise processing described above with reference to Figures 4-8 (for example the first correlation computation and update processing).

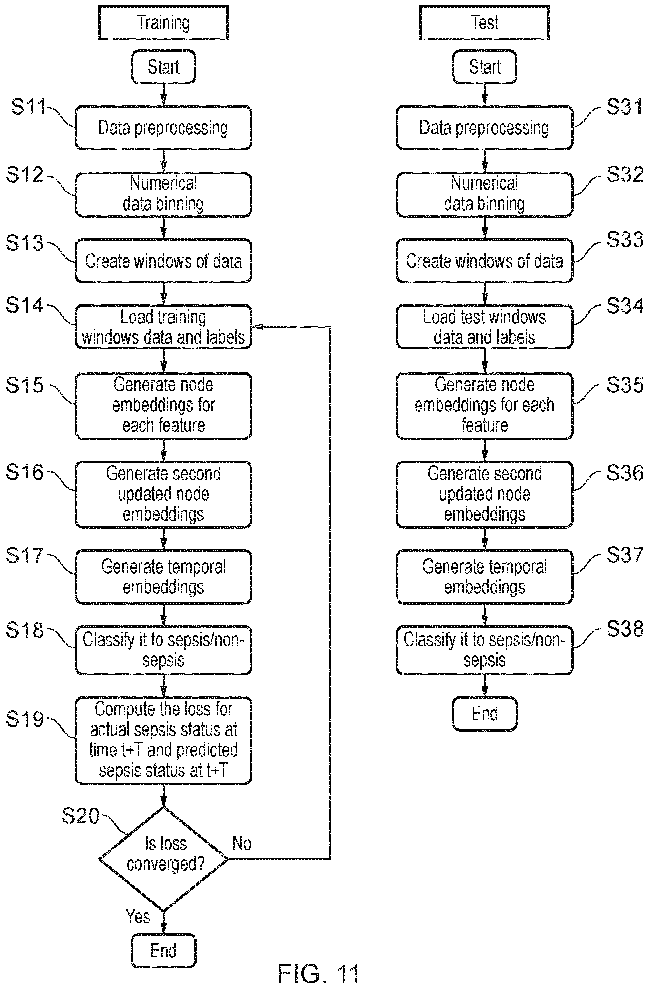

Figure 11 is a schematic diagram of a training process according to an implementation and a test process according to an implementation. The training process comprises steps S11-S20. Step S11 comprises data preprocessing. Step S12 comprises numerical data binning. Numerical data binning is a method to generate the high dimensional encodings based on the input data. Step S11 comprises creating windows of data. Step S13 comprises creating data windows which comprises generating a constant length time series, i.e., making every time series have "T" number of time steps.

Step S14 comprises loading data windows and labels as training data. The labels comprise (as indicated in step S19) a "ground truth" classification of the patient having sepsis at time t+T. Step S15 comprises generating a node embedding (E') for each data point. Step S15 may be considered to comprise multiplying the high dimensional features/encoding with weight vectors. Step S16 comprises generating the cross-correlational embeddings (i.e. generating the first correlations and updating the node embeddings based thereon). Step S17 comprises generating temporal embeddings (i.e. generating the second correlations based on the first updated node embeddings and updating the first updated node embeddings based thereon to generate the temporally updated embeddings.

Step S18 comprises classifying the training data as sepsis or non-sepsis, i.e. generating the prediction indicating whether the patient will have or not have sepsis at time t+T (some predefined time in the future). Step S19 comprises comparing the prediction to the label indicating a "ground truth" sepsis/non-sepsis classification and computing the loss therebetween. The prediction indicated by the label may be referred to as a training prediction. The training process in an implementation comprises using cross-entropy loss or focal loss. Weighted cross-entropy loss or logarithmic loss may be used. What is used may depend on the amount of class imbalance in the dataset, for example.

Although not shown in Figure 11, the training process comprises a step of adjusting at least one network weight used in the modified attention mechanism (to generate the short range temporal correlations), the self-attention mechanism (to generate the spatial correlations), the RNN (to generate the second correlations), and the NN (used to predict the classification) based on the loss (i.e. a difference between the generated prediction/diagnosis and the training prediction/diagnosis corresponding to the training data, to bring the generated prediction to or towards the training prediction). For example, all the network weights in the modified attention and self-attention mechanisms, the RNN, and the NN are adjusted.

Step S20 comprises determining whether the loss is converged. If yes then the method ends. If no then the method proceeds to step S14 and more training data is loaded. The training process is iterated in this way. For example, it may be determined whether the loss is below a threshold or has been below a threshold for a predetermined number of iterations. Alternatively or additionally, step S20 may comprise determining whether the maximum number of epochs has been reached.

This may be determined in addition to the loss convergence - i.e. the method may end if either determination is true ("yes"), that is, if either the maximum number of epochs has been reached or if the loss is determined to be converging/have converged.

The training process may not be considered to comprises any of steps S11-S14 and any of the steps may be considered already carried out as part of a pre-training process. A method of training a system for sepsis prediction may comprise using input data related to different patients per iteration. Any of the steps S15-S18 may comprise any of the corresponding processing described above with reference to Figures 4-8 and 10. A training process may comprise performing the prediction process described with reference to Figure 10, or the processing described with reference to Figures 4-8, followed by a loss determination, a weight adjustment, and a step corresponding to step S20, and multiple iterations thereof using training data with corresponding training predictions (ground truth labels).

The test process comprises steps S31-S38. Steps S31-S38 correspond with steps S11-S18, except test data is used rather than training data. The test process ends with a prediction/diagnosis/classification of sepsis/non-sepsis in step S38. The considerations described with respect to steps S11-S18 similarly apply to steps S31-S38, respectively.

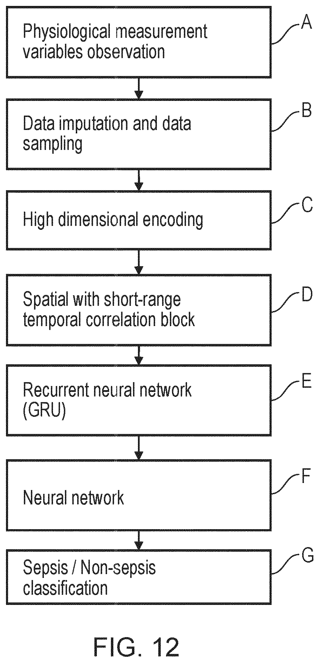

Figure 12 is a schematic diagram illustrating blocks representing a prediction process according to an implementation. The process comprises in step A receiving input data (values of physiological measurement variables, e.g. vital signs) and in step B performing data imputation and sampling. In step C a high-dimensional encoding is generated based on the input data (e.g. using data binning). in step D first correlations are generated using the spatial with short range temporal correlation learning mechanism (e.g. the processing of the correlation block 43) and updated node embeddings generated based thereon, and then in step E temporally updated embeddings are generated using a GRU RNN. In step F an NN (fully connected layer) is used to generate the sepsis/non-sepsis classification/prediction/diagnosis, which may be considered output as a classification in step G.

An implementation of the neural network is described below but is not essential. A neural network can be composed of 2 fully connected layers and 1 fully connected classification layer, which means it is a type of neural network that has 3 layers of neurons. The first 2 layers are fully connected, meaning that every neuron in one layer is connected to every neuron in the next layer. The third layer is a fully connected classification layer, meaning that it has a number of output neurons equal to the number of classes in the problem (in this case it is 2). The input to the neural network is a vector of real numbers (in this case input dimension is [1 x 128]). Each layer can be represented mathematically as follows:

Let h be the input data point (dimension [1 x 128]), and let y be the output label (0 or 1). Each fully connected layer can be represented by a weight matrix W and a bias vector b. The output of the layer is then given by:

$$z = W * x + b$$

where z is the output of the layer.

The output z is then passed through a non-linear activation function f. As an activation function, there are a number of options such as ReLU, Leaky ReLU etc. These may be used by the intermediate layer. The most common activation function for classification tasks (last layer) is the softmax function or sigmoid function. The softmax function takes a vector of real numbers as input and outputs a vector of probabilities. The probabilities represent the probability of each label being assigned to the input data point. The label with the highest probability is then chosen as the output label. It is noted that 2 or more fully connected layers may be used for the NN.

An implementation of a method of training used to train the NN is as follows. The architecture that may be used herein is end-to-end architecture which takes the input as the second updated embeddings (h) and produces a vector of probabilities as output from classification layer, wherein the loss is calculated using a loss function (e.g. cross-entropy loss, focal loss etc.). The loss function is then used to train the model using an optimization algorithm. The optimization algorithm tries to find the values of the model's parameters that minimize the loss (stochastic gradient descent, Adam, Adagrad etc).

The use of vital signs data as the physiological measurement variables is not essential. A GRU RNN is not essential - an RNN-based network that is not necessarily with a GRU RNN is used in some implementations.

A data binning method may be carried out as follows, using a value of temperature as an example. The value is assigned to a bin, for example bin number 6 (the bins represent intervals of temperature values, e.g. bin 6 may represent the interval 36-36.3 degrees Celsius). Then a high dimensional encoding for that value is an array comprising 5 zeroes (representing bins 1-5), a normalized (between 0 and 1) value corresponding to the actual temperature value, and then a number of 1s corresponding to how many other bins exist above bin 6. For example if there are 10 bins the high dimensional encoding may look like: "00000 y 1 1 1 1", where y is the normalized temperature value. Data binning is merely one way of generating high dimensional encodings.

The second correlations may be referred to as long range temporal correlations. The output of an RNN may comprise the output of the last GRU block.

Any of the above prediction processes may be repeated so that an iteration is carried out every time period, e.g. every hour or half-hour, or using irregular intervals of time. Up-to-data input data (vital signs data) may be used each iteration. That is, each successive iteration may use more (and more recent) input data. This may be considered monitoring the patient for sepsis risk. A prediction/classification of sepsis is a diagnosis of sepsis based on physiological measurements of the patient which may be obtained using at least one sensor. Graphs/networks may be stored in the form of linked data nodes.

As described above, a training process/method is disclosed herein which comprises performing any prediction process described above for multiple iterations and performing weight adjustment based on the loss each iteration.

According to an aspect there is disclosed herein a method to predict sepsis, before actual sepsis onset, in an ICU setting, using physiological data of the patient recorded at regular or irregular time intervals (e.g. using hourly recorded physiological data of the patient). Improved accuracy compared to other methods is achieved by modelling spatial correlation/spatial and temporal structure of patients' physiological parameters/vitals with graph-based network and RNN. Sepsis prediction is high in demand as it saves human lives and hospital resources. Finding the correlation between vitals in spatial and temporal directions simultaneously is difficult and until now not fully explored.

An objective of aspects disclosed herein may be considered an early sepsis prediction before sepsis onset in an ICU setting, and this is achieved by exploiting the cross-correlation and temporal structure of the patients' physiological data.

Limitations of existing sepsis prediction models include the fact that they don't consider the correlational structure of patients various physiological and other body parameters during the prediction of sepsis, nor the fact that their cross-correlation changes as the disease progresses with time. Furthermore, the spatial cross-correlation patterns in comparative method 1 do not include the cross-correlation patterns between features of different (consecutive) time steps (short range temporal correlations). In addition, the number of timestamps (in sepsis prediction based on physiological parameters, e.g. vital signs) is not necessarily fixed but the convolutions employed in comparative method 1 need a fixed input size. Aspects disclosed herein employ a memory learning mechanism, not merely a simple aggregation over time as in comparative method 1. Moreover, more recent timeframes are more important (in sepsis prediction), which is not captured in comparative method 1.

The spatial cross-correlation patterns in comparative method 2 do not include temporal cross-correlation patterns between features of different (consecutive) time steps (short range temporal correlations). If use of comparative method 2 was attempted for physiological parameters-based sepsis prediction, each layer would have different vital nodes information combinations so edge weights cannot be computed based on previous layer edge weight. Comparative method 2 merely employs time-aggregation of attention weights between nodes, whereas in contrast aspects disclosed herein model attention between nodes at every time-frame and retain the latest evolving relation between nodes. Hence an RNN is used to obtain second (long range temporal) correlations.

Aspects disclosed herein are capable of predicting sepsis a few hours before sepsis onset by using only patient's physiological time series data. Improved sepsis prediction accuracy with only vitals data is achieved. Aspects may help the building of multi-variate time-series models for sepsis prediction in an ICU setting. General methods to predict sepsis including modelling via RNNs, time domain CNNs, and classic machine learning methods do not consider interdependencies between physiological parameters, for example.

Aspects disclosed herein may include graph-based spatio (cross-correlation)-temporal network and learning strategy for sepsis prediction; early sepsis prediction using physiological parameters or only vital signs; inclusion of the short-range temporal cross-correlation embedding in estimation of spatial cross-correlation embedding; unification of the cross-correlation structure with temporal structure to exploit the spatio-temporal aspect of the data.

Where processing is described as being performed by/using specific architecture, this architecture is not essential and other architecture may be used to perform the same processing.

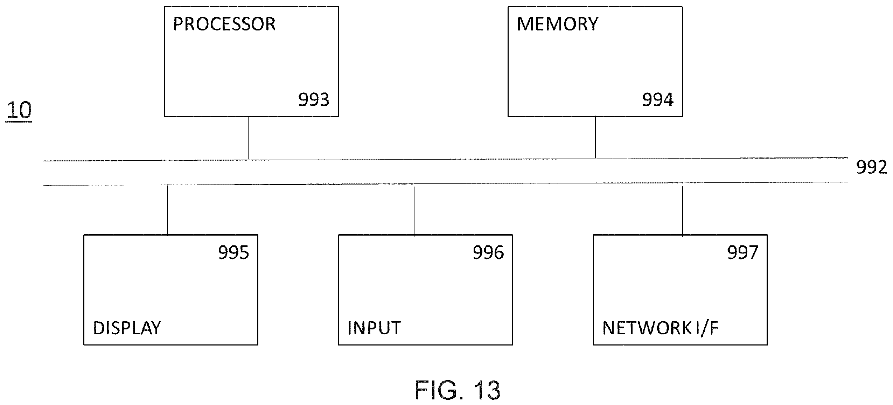

Figure 13 is a block diagram of an information processing apparatus 10 or a computing device 10, such as a data storage server, which embodies the present invention, and which may be used to implement some or all of the operations of a method embodying the present invention, and perform some or all of the tasks of apparatus of an embodiment. The computing device 10 may be used to implement any of the method steps described above, e.g. any of steps S51-S54, S11-S20, and S31-S38 and/or any processes/processing described above.

The computing device 10 comprises a processor 993 and memory 994. Optionally, the computing device also includes a network interface 997 for communication with other such computing devices, for example with other computing devices of invention embodiments. Optionally, the computing device also includes one or more input mechanisms such as keyboard and mouse 996, and a display unit such as one or more monitors 995. These elements may facilitate user interaction. The components are connectable to one another via a bus 992.

The memory 994 may include a computer readable medium, which term may refer to a single medium or multiple media (e.g., a centralized or distributed database and/or associated caches and servers) configured to carry computer-executable instructions. Computer-executable instructions may include, for example, instructions and data accessible by and causing a computer (e.g., one or more processors) to perform one or more functions or operations. For example, the computer-executable instructions may include those instructions for implementing a method disclosed herein, or any method steps disclosed herein, e.g. any of steps S51-S54, S11-S20, and S31-S38 and/or any processes/processing described above. Thus, the term "computer-readable storage medium" may also include any medium that is capable of storing, encoding or carrying a set of instructions for execution by the machine and that cause the machine to perform any one or more of the method steps of the present disclosure. The term "computer-readable storage medium" may accordingly be taken to include, but not be limited to, solid-state memories, optical media and magnetic media. By way of example, and not limitation, such computer-readable media may include non-transitory computer-readable storage media, including Random Access Memory (RAM), Read-Only Memory (ROM), Electrically Erasable Programmable Read-Only Memory (EEPROM), Compact Disc Read-Only Memory (CD-ROM) or other optical disk storage, magnetic disk storage or other magnetic storage devices, flash memory devices (e.g., solid state memory devices).

The processor 993 is configured to control the computing device and execute processing operations, for example executing computer program code stored in the memory 994 to implement any of the method steps described herein. The memory 994 stores data being read and written by the processor 993 and may store weights and/or input data and/or equations and/or training data and/or labels and/or nodes and weights of networks and/or other data, described above, and/or programs for executing any of the method steps/processes described above. As referred to herein, a processor may include one or more general-purpose processing devices such as a microprocessor, central processing unit, or the like. The processor may include a complex instruction set computing (CISC) microprocessor, reduced instruction set computing (RISC) microprocessor, very long instruction word (VLIW) microprocessor, or a processor implementing other instruction sets or processors implementing a combination of instruction sets. The processor may also include one or more special-purpose processing devices such as an application specific integrated circuit (ASIC), a field programmable gate array (FPGA), a digital signal processor (DSP), network processor, or the like. In one or more embodiments, a processor is configured to execute instructions for performing the operations and operations discussed herein. The processor 993 may be considered to comprise any of the modules described above. Any operations described as being implemented by a module may be implemented as a method by a computer and e.g. by the processor 993.

The display unit 995 may display a representation of data stored by the computing device, such as a prediction and/or data and/or a representation of networks and/or GUI windows and/or interactive representations enabling a user to interact with the apparatus 10 by e.g. drag and drop or selection interaction, and/or any other output described above, and may also display a cursor and dialog boxes and screens enabling interaction between a user and the programs and data stored on the computing device. The input mechanisms 996 may enable a user to input data and instructions to the computing device, such as enabling a user to input any user input described above.

The network interface (network I/F) 997 may be connected to a network, such as the Internet, and is connectable to other such computing devices via the network. The network I/F 997 may control data input/output from/to other apparatus via the network. Other peripheral devices such as microphone, speakers, printer, power supply unit, fan, case, scanner, trackball etc may be included in the computing device.

Methods embodying the present invention may be carried out on a computing device/apparatus 10 such as that illustrated in Figure 13. Such a computing device need not have every component illustrated in Figure 13, and may be composed of a subset of those components. For example, the apparatus 10 may comprise the processor 993 and the memory 994 connected to the processor 993. Or the apparatus 10 may comprise the processor 993, the memory 994 connected to the processor 993, and the display 995. A method embodying the present invention may be carried out by a single computing device in communication with one or more data storage servers via a network. The computing device may be a data storage itself storing at least a portion of the data.

A method embodying the present invention may be carried out by a plurality of computing devices operating in cooperation with one another. One or more of the plurality of computing devices may be a data storage server storing at least a portion of the data.

The invention may be implemented in digital electronic circuitry, or in computer hardware, firmware, software, or in combinations of them. The invention may be implemented as a computer program or computer program product, i.e., a computer program tangibly embodied in a non-transitory information carrier, e.g., in a machine-readable storage device, or in a propagated signal, for execution by, or to control the operation of, one or more hardware modules.

A computer program may be in the form of a stand-alone program, a computer program portion or more than one computer program and may be written in any form of programming language, including compiled or interpreted languages, and it may be deployed in any form, including as a stand-alone program or as a module, component, subroutine, or other unit suitable for use in a data processing environment. A computer program may be deployed to be executed on one module or on multiple modules at one site or distributed across multiple sites and interconnected by a communication network.

Method steps of the invention may be performed by one or more programmable processors executing a computer program to perform functions of the invention by operating on input data and generating output. Apparatus of the invention may be implemented as programmed hardware or as special purpose logic circuitry, including e.g., an FPGA (field programmable gate array) or an ASIC (applicationspecific integrated circuit).

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for executing instructions coupled to one or more memory devices for storing instructions and data.

The above-described embodiments of the present invention may advantageously be used independently of any other of the embodiments or in any feasible combination with one or more others of the embodiments.

All examples and conditional language provided herein are intended for the pedagogical purposes of aiding the reader in understanding the invention and the concepts contributed by the inventor to further the art, and are not to be construed as limitations to such specifically recited examples and conditions, nor does the organization of such examples in the specification relate to a showing of the superiority and inferiority of the invention.

**Claims**

1. A computer-implemented method comprising:
   performing a prediction process, the prediction process comprising:

   based on input data comprising values of physiological measurement variables of a patient over a time period, computing (S51) first correlations in the input data, the computing comprising computing short range temporal correlations between values of physiological measurement variables at consecutive time steps using an attention-based mechanism and computing spatial correlations between values of different physiological measurement variables at a same time step using a self-attention mechanism;
   generating (S52) first updated node embeddings based on the input data and the first correlations, each node corresponding to a physiological measurement variable at a time step;
   using a recurrent neural network, RNN, generating long-range temporal correlations between the first updated node embeddings and updating (S53) the first updated node embeddings based on the long-range temporal correlations to generate temporally updated embeddings; and
   based on the temporally updated embeddings and using (S54) a neural network, NN, generating a prediction indicating whether the patient will have sepsis.

2. The computer-implemented method as claimed in claim 1, wherein the physiological measurement variables comprise at least two of:

   heart rate;
   oxygen saturation;
   temperature;
   blood pressure;
   respiration rate;
   end-tidal carbon dioxide;
   blood sugar;
   Base Excess,
   bicarbonate, HCO3, level;
   fibrinogen level,
   platelets level;
   Fraction of Inspired Oxygen level
   pH level;
   Partial Pressure of Carbon Dioxide;
   oxygen saturation of arterial blood;
   Aspartate aminotransferase level;
   Blood urea nitrogen;
   Alkaline phosphatase level;
   Calcium level;
   Chloride level;
   Creatinine level;

Direct Bilirubin level;
Glucose level;
Lactate level;
Magnesium level;
Phosphate level;
Potassium level;
Total Bilirubin level;
Troponin I level;
Haematocrit level;
Haemoglobin level;
Partial thromboplastin level; and
White blood cell level.

3. The computer-implemented method as claimed in claim 1 or claim 2, wherein computing the short range temporal correlations for a value of a physiological measurement variable at a time step comprises computing correlations between that value and the value of each physiological measurement variable at the previous time step.

4. The computer-implemented method as claimed any of claims 1-3, wherein the prediction process comprises:

    generating multi-dimensional feature encodings based on the input data; and
    generating initial node embeddings by multiplying the multi-dimensional feature encodings with weight vectors,
    wherein the computing the first correlations comprises computing the first correlations based on the initial node embeddings.

5. The computer-implemented method as claimed in claim 4, wherein:

    computing the short range temporal correlations for the values corresponding to the second time step in the order of time steps comprises computing correlations between each of the initial node embeddings corresponding to the second time step and each of the initial node embeddings corresponding to the first time step; and
    computing the short range temporal correlations for the values corresponding to each of the third and subsequent time steps in the order of time steps comprises computing correlations between each of the initial node embeddings corresponding to the time step concerned and each of the first updated node embeddings corresponding to the preceding time step.

6. The computer-implemented method as claimed in claim 5, wherein the generating the first updated node embeddings comprises updating each intermediate node embedding based on its correlation with each other intermediate node embedding corresponding to the same time step.

7. The computer-implemented method as claimed in any of the preceding claims, comprising performing a training process, the training process comprising:

    performing the prediction process using training data corresponding to a training patient as the input data;
    adjusting at least one network weight used in the attention-based mechanism, the self-attention mechanism, the RNN, and the NN based on a difference between the generated prediction and a training prediction corresponding to the training data.

8. The computer-implemented method as claimed in claim 7, comprising, after performing the training process, performing the prediction process using target input data of a target patient to generate a target prediction.

9. The computer-implemented method as claimed in any of the preceding claims, wherein generating the multi-dimensional feature encodings comprises, for each physiological measurement variable, performing a data binning method on the values concerned and generating, as the multi-dimensional feature encodings, a feature vector for each value.

10. The computer-implemented method as claimed in any of the preceding claims, wherein generating the spatial correlations comprises using a key-query-value self-attention mechanism.

11. The computer-implemented method as claimed in any of the preceding claims, wherein generating the short range temporal correlations comprises using a key-query-value attention-based mechanism.

12. The computer-implemented method as claimed in any of the preceding claims, wherein the prediction process comprises repeatedly generating said first correlations and updating the node embeddings concerned, comprising, for each subsequent iteration, starting with the first updated node embeddings of the previous iteration in place of the initial node embeddings.

13. A computer program which, when run on a computer, causes the computer to carry out a method comprising:
performing a prediction process, the prediction process comprising:

    based on input data comprising values of physiological measurement variables of a patient over a time period, computing (S51) first correlations in the input data, the computing comprising computing short range temporal correlations between values of physiological measurement variables at consecutive time steps using an attention-based mechanism and computing spatial correlations between values of different physiological measurement variables at a same time step using a self-attention mechanism; generating (S52) first updated node embeddings based on the input data and the first correlations, each node corresponding to a physiological measurement variable at a time step; using a recurrent neural network, RNN, generating long-range temporal correlations between the first updated node embeddings and updating (S53) the first updated node embedding based on the long-range temporal correlations to generate temporally updated embeddings; and

based on the temporally updated embeddings and using (S54) a neural network, NN, generating a prediction indicating whether the patient will have sepsis.

14. An information processing apparatus (10) comprising a memory (994) and a processor (993) connected to the memory (994), wherein the processor (993) is configured to:

perform a prediction process, the prediction process comprising:

based on input data comprising values of physiological measurement variables of a patient over a time period, computing first correlations in the input data, comprising computing short range temporal correlations between values of physiological measurement variables at consecutive time steps using an attention-based mechanism and computing spatial correlations between values of different physiological measurement variables at a same time step using a self-attention mechanism; generating first updated node embeddings based on the input data and the first correlations, each node corresponding to a physiological measurement variable at a time step; using a recurrent neural network, RNN, generating long-range temporal correlations between the first updated node embeddings and updating the first updated node embedding based on the long-range temporal correlations to generate temporally updated embeddings; and based on the temporally updated embeddings and using a neural network, NN, generating a prediction indicating whether the patient will have sepsis.

## Patentansprüche

1. Computerimplementiertes Verfahren, umfassend: Durchführen eines Vorhersageprozesses, wobei der Vorhersageprozess umfasst:

basierend auf Eingabedaten, die Werte physiologischer Messvariablen eines Patienten über einen Zeitraum umfassen, Berechnen (S51) erster Korrelationen in den Eingabedaten, wobei das Berechnen das Berechnen kurzreichweitiger zeitlicher Korrelationen zwischen Werten physiologischer Messvariablen an aufeinanderfolgenden Zeitschritten unter Verwendung eines aufmerksamkeitsbasierten Mechanismus und das Berechnen räumlicher Korrelationen zwischen Werten verschiedener physiologischer Messvariablen an demselben Zeitschritt unter Verwendung eines Selbstaufmerksamkeitsmechanismus umfasst; Erzeugen (S52) erster aktualisierter Knoteneinbettungen basierend auf den Eingabedaten und den ersten Korrelationen, wobei jeder Knoten einer physiologischen Messvariablen an einem Zeitschritt entspricht; unter Verwendung eines rekurrenten neuronalen Netzwerks (RNN - recurrent neural network), Erzeugen weitreichender zeitlicher Korrelationen zwischen den ersten aktualisierten Knoteneinbettungen und Aktualisieren (S53) der ersten aktualisierten Knoteneinbettungen basierend auf den weitreichenden zeitlichen Korrelationen, um zeitlich aktualisierte Einbettungen zu erzeugen; und basierend auf den zeitlich aktualisierten Einbettungen und unter Verwendung (S54) eines neuronalen Netzwerks (NN - neural network), Erzeugen einer Vorhersage, die angibt, ob der Patient Sepsis haben wird.

2. Computerimplementiertes Verfahren nach Anspruch 1, wobei die physiologischen Messvariablen mindestens zwei der folgenden umfassen:

Herzfrequenz;
Sauerstoffsättigung;
Temperatur;
Blutdruck;
Atemfrequenz;
endtidales Kohlendioxid;
Blutzucker;
Basenüberschuss,
Bicarbonat, HCO3, Spiegel;
Fibrinogenspiegel,
Thrombozytenspiegel;
Spiegel der eingeatmeten Sauerstofffraktion
pH-Wert;
Kohlendioxidpartialdruck;

Sauerstoffsättigung des arteriellen Blutes;
Aspartataminotransferase-Spiegel;
Blutharnstoffstickstoff;
Alkalische-Phosphatase-Spiegel;
Calciumspiegel;
Chloridspiegel;
Kreatininspiegel;
Direkter-Bilirubin-Spiegel;
Glukosespiegel;
Lactatspiegel;
Magnesiumspiegel;
Phosphatspiegel;
Kaliumspiegel;
Gesamtbilirubinspiegel;
Troponin-I-Spiegel;
Hämatokritspiegel;
Hämoglobinspiegel;
Partieller Thromboplastinspiegel; und
Weißblutkörperchenspiegel.

3. Computerimplementiertes Verfahren nach Anspruch 1 oder Anspruch 2, wobei das Berechnen der kurzreichweitigen zeitlichen Korrelationen für einen Wert einer physiologischen Messvariable bei einem Zeitschritt das Berechnen von Korrelationen zwischen diesem Wert und dem Wert jeder physiologischen Messvariable beim vorherigen Zeitschritt umfasst.

4. Computerimplementiertes Verfahren nach einem der Ansprüche 1-3, wobei der Vorhersageprozess umfasst:

Erzeugen von mehrdimensionalen Merkmalscodierungen basierend auf den Eingabedaten; und
Erzeugen von anfänglichen Knoteneinbettungen durch Multiplizieren der mehrdimensionalen Merkmalscodierungen mit Gewichtsvektoren,
wobei das Berechnen der ersten Korrelationen das Berechnen der ersten Korrelationen basierend auf den anfänglichen Knoteneinbettungen umfasst.

5. Computerimplementiertes Verfahren nach Anspruch 4, wobei:

das Berechnen der kurzreichweitigen zeitlichen Korrelationen für die Werte, die dem zweiten Zeitschritt in der Reihenfolge der Zeitschritte entsprechen, das Berechnen von Korrelationen zwischen jeder der anfänglichen Knoteneinbettungen, die dem zweiten Zeitschritt entsprechen, und jeder der anfänglichen Knoteneinbettungen, die dem ersten Zeitschritt entsprechen, umfasst; und
das Berechnen der kurzreichweitigen zeitlichen Korrelationen für die Werte, die jedem des dritten und der nachfolgenden Zeitschritte in der Reihenfolge der Zeitschritte entsprechen, das Berechnen von Korrelationen zwischen jeder der anfänglichen Knoteneinbettungen, die dem betreffenden Zeitschritt entsprechen, und jeder der ersten aktualisierten Knoteneinbettungen, die dem vorhergehenden Zeitschritt entsprechen, umfasst.

6. Computerimplementiertes Verfahren nach Anspruch 5, wobei das Erzeugen der ersten aktualisierten Knoteneinbettungen das Aktualisieren jeder intermediären Knoteneinbettung basierend auf ihrer Korrelation mit jeder anderen intermediären Knoteneinbettung, die demselben Zeitschritt entspricht, umfasst.

7. Computerimplementiertes Verfahren nach einem der vorhergehenden Ansprüche, umfassend das Durchführen eines Trainingsprozesses, wobei der Trainingsprozess umfasst:

Durchführen des Vorhersageprozesses unter Verwendung von Trainingsdaten, die einem Trainingspatienten entsprechen, als die Eingabedaten;
Anpassen mindestens eines Netzwerkgewichts, das in dem aufmerksamkeitsbasierten Mechanismus, dem Selbstaufmerksamkeitsmechanismus, dem RNN und dem NN verwendet wird, basierend auf einer Differenz zwischen der erzeugten Vorhersage und einer Trainingsvorhersage, die den Trainingsdaten entspricht.

8. Computerimplementiertes Verfahren nach Anspruch 7, umfassend, nach dem Durchführen des Trainingsprozesses, das Durchführen des Vorhersageprozesses unter Verwendung von Ziel-Eingabedaten eines Zielpatienten, um eine Zielvorhersage zu erzeugen.

9. Computerimplementiertes Verfahren nach einem der vorhergehenden Ansprüche, wobei das Erzeugen der mehrdimensionalen Merkmalscodierungen umfasst, für jede physiologische Messvariable, das Durchführen eines Datenbinning-Verfahrens an den betreffenden Werten und das Erzeugen, als die mehrdimensionalen Merkmalscodierungen, eines Merkmalsvektors für jeden Wert.

10. Computerimplementiertes Verfahren nach einem der vorhergehenden Ansprüche, wobei das Erzeugen der räumlichen Korrelationen die Verwendung eines Schlüssel-Anfrage-Wert-Selbstaufmerksamkeitsmechanismus umfasst.

11. Computerimplementiertes Verfahren nach einem der vorhergehenden Ansprüche, wobei das Erzeugen der kurzreichweitigen zeitlichen Korrelationen die Verwendung eines Schlüssel-Anfrage-Wert-aufmerksamkeitsbasierten Mechanismus umfasst.

12. Computerimplementiertes Verfahren nach einem der vorhergehenden Ansprüche, wobei der Vorhersageprozess das wiederholte Erzeugen der ersten Korrelationen und das Aktualisieren der betreffenden Knoteneinbettungen umfasst, umfassend, für jede nachfolgende Iteration, das Beginnen mit den ersten aktualisierten Knoteneinbettungen der vorherigen Iteration anstelle der anfänglichen Knoteneinbettungen.

13. Computerprogramm, das, wenn es auf einem Computer ausgeführt wird, den Computer veranlasst, ein Verfahren durchzuführen, umfassend:
Durchführen eines Vorhersageprozesses, wobei der Vorhersageprozess umfasst:

basierend auf Eingabedaten, die Werte von physiologischen Messvariablen eines Patienten über einen Zeitraum umfassen, Berechnen (S51) von ersten Korrelationen in den Eingabedaten,
wobei das Berechnen das Berechnen von kurzreichweitigen zeitlichen Korrelationen zwischen Werten von physiologischen Messvariablen bei aufeinanderfolgenden Zeitschritten unter Verwendung eines aufmerksamkeitsbasierten Mechanismus und das Berechnen von räumlichen Korrelationen zwischen Werten verschiedener physiologischer Messvariablen bei demselben Zeitschritt unter Verwendung eines Selbstaufmerksamkeitsmechanismus umfasst;
Erzeugen (S52) von ersten aktualisierten Knoteneinbettungen basierend auf den Eingabedaten und den ersten Korrelationen, wobei jeder Knoten einer physiologischen Messvariable bei einem Zeitschritt entspricht;
unter Verwendung eines rekurrenten neuronalen Netzwerks, RNN, Erzeugen von weitreichenden zeitlichen Korrelationen zwischen den ersten aktualisierten Knoteneinbettungen und Aktualisieren (S53) der ersten aktualisierten Knoteneinbettung basierend auf den weitreichenden zeitlichen Korrelationen, um zeitlich aktualisierte Einbettungen zu erzeugen; und
basierend auf den zeitlich aktualisierten Einbettungen und unter Verwendung (S54) eines neuronalen Netzwerks, NN, Erzeugen einer Vorhersage, die angibt, ob der Patient Sepsis haben wird.

14. Informationsverarbeitungseinrichtung (10), umfassend einen Speicher (994) und einen Prozessor (993), der mit dem Speicher (994) verbunden ist, wobei der Prozessor (993) konfiguriert ist zum:
Durchführen eines Vorhersageprozesses, wobei der Vorhersageprozess umfasst:

basierend auf Eingabedaten, die Werte von physiologischen Messvariablen eines Patienten über einen Zeitraum umfassen, Berechnen von ersten Korrelationen in den Eingabedaten, umfassend das Berechnen von kurzreichweitigen zeitlichen Korrelationen zwischen Werten von physiologischen Messvariablen bei aufeinanderfolgenden Zeitschritten unter Verwendung eines aufmerksamkeitsbasierten Mechanismus und das Berechnen von räumlichen Korrelationen zwischen Werten verschiedener physiologischer Messvariablen bei demselben Zeitschritt unter Verwendung eines Selbstaufmerksamkeitsmechanismus;
Erzeugen von ersten aktualisierten Knoteneinbettungen basierend auf den Eingabedaten und den ersten Korrelationen, wobei jeder Knoten einer physiologischen Messvariable bei einem Zeitschritt entspricht;
unter Verwendung eines rekurrenten neuronalen Netzwerks, RNN, Erzeugen von weitreichenden zeitlichen Korrelationen zwischen den ersten aktualisierten Knoteneinbettungen und Aktualisieren der ersten aktualisierten Knoteneinbettung basierend auf den weitreichenden zeitlichen Korrelationen, um zeitlich aktualisierte Einbettungen zu erzeugen; und
basierend auf den zeitlich aktualisierten Einbettungen und unter Verwendung eines neuronalen Netzwerks, NN, Erzeugen einer Vorhersage, die angibt, ob der Patient Sepsis haben wird.

**Revendications**

1. Procédé mis en œuvre par ordinateur, comprenant :
la réalisation d'un processus de prédiction, le processus de prédiction comprenant :

sur la base de données d'entrée comprenant des valeurs de variables de mesure physiologiques d'un patient sur une période de temps, le calcul (S51) de premières corrélations dans les données d'entrée, le calcul comprenant le calcul de corrélations temporelles à court terme entre les valeurs de variables de mesure physiologiques à des étapes temporelles consécutives à l'aide d'un mécanisme basé sur l'attention et le calcul de corrélations spatiales entre les valeurs de différentes variables de mesure physiologiques à une même étape temporelle à l'aide d'un mécanisme d'auto-attention ;
la génération (S52) de premières intégrations de nœud mises à jour sur la base des données d'entrée et des premières corrélations, chaque nœud correspondant à une variable de mesure physiologique à une étape temporelle ;

à l'aide d'un réseau neuronal récurrent, RNN, la génération de corrélations temporelles à long terme entre les premières intégrations de nœud mises à jour et la mise à jour (S53) des premières intégrations de nœud mises à jour sur la base des corrélations temporelles à long terme pour générer des intégrations mises à jour temporellement ; et

sur la base des intégrations mises à jour temporellement et à l'aide (S54) d'un réseau neuronal, NN, la génération d'une prédiction indiquant si le patient aura une septicémie.

2. Procédé mis en œuvre par ordinateur selon la revendication 1, dans lequel les variables de mesure physiologiques comprennent au moins deux des éléments suivants :

une fréquence cardiaque ;
une saturation en oxygène ;
une température ;
une pression artérielle ;
un rythme respiratoire ;
un dioxyde de carbone en fin d'expiration ;
une glycémie ;
un excès de base,
un niveau de bicarbonate, HCO3 ;
un niveau de fibrinogène,
un niveau de plaquettes ;
un niveau de fraction d'oxygène inspiré
un niveau de pH ;
une pression partielle de dioxyde de carbone ;
une saturation en oxygène de sang artériel ;
un niveau d'aspartate aminotransférase ;
un azote uréique sanguin ;
un niveau de phosphatase alcaline ;
un niveau de calcium ;
un niveau de chlorure ;
un niveau de créatinine ;
un niveau de bilirubine directe ;
un niveau de glucose ;
un niveau de lactate ;
un niveau de magnésium ;
un niveau de phosphate ;
un niveau de potassium ;
un niveau de bilirubine totale ;
un niveau de troponine I ;
un niveau d'hématocrite ;
un niveau d'hémoglobine ;
un niveau de thromboplastine partielle ; et
un niveau de globules blancs.

3. Procédé mis en œuvre par ordinateur selon la revendication 1 ou la revendication 2, dans lequel le calcul des corrélations temporelles à court terme pour une valeur d'une variable de mesure physiologique à une étape temporelle comprend le calcul de corrélations entre cette valeur et la valeur de chaque variable de mesure physiologique à l'étape temporelle précédente.

4. Procédé mis en œuvre par ordinateur selon l'une quelconque des revendications 1 à 3, dans lequel le processus de prédiction comprend :

la génération d'encodages de caractéristique multidimensionnels sur la base des données d'entrée ; et
la génération d'intégrations de nœud initiales en multipliant les encodages de caractéristique multidimensionnels par des vecteurs de poids, dans lequel le calcul des premières corrélations comprend le calcul des premières corrélations sur la base des intégrations de nœud initiales.

5. Procédé mis en œuvre par ordinateur selon la revendication 4, dans lequel :

le calcul des corrélations temporelles à court terme pour les valeurs correspondant à la deuxième étape temporelle, dans l'ordre des étapes temporelles, comprend le calcul de corrélations entre chacune des intégrations de nœud initiales correspondant à la deuxième étape temporelle et chacune des intégrations de nœud initiales correspondant à la première étape temporelle ; et
le calcul des corrélations temporelles à court terme pour les valeurs correspondant à chacune de la troisième étape et des étapes temporelles ultérieures, dans l'ordre des étapes temporelles, comprend le calcul de corrélations entre chacune des intégrations de nœud initiales correspondant à l'étape temporelle concernée et chacune des premières intégrations de nœud mises à jour correspondant à l'étape temporelle précédente.

6. Procédé mis en œuvre par ordinateur selon la revendication 5, dans lequel la génération des premières intégrations de nœud mises à jour comprend la mise à jour de chaque intégration de nœud intermédiaire sur la base de sa corrélation avec chaque autre intégration de nœud intermédiaire correspondant à la même étape temporelle.

7. Procédé mis en œuvre par ordinateur selon l'une quelconque des revendications précédentes, comprenant la réalisation d'un processus d'entraînement, le processus d'entraînement comprenant :

la réalisation du processus de prédiction à l'aide, en tant que donnée d'entrée, de données d'entraînement correspondant à un patient d'entraînement ;

l'ajustement d'au moins un poids de réseau utilisé dans le mécanisme basé sur l'attention, le mécanisme d'auto-attention, le RNN et le NN sur la base d'une différence entre la prédiction générée et une prédiction d'entraînement correspondant aux données d'entraînement.

8.  Procédé mis en œuvre par ordinateur selon la revendication 7, comprenant, après la réalisation du processus d'entraînement, la réalisation du processus de prédiction à l'aide de données d'entrée cibles d'un patient cible pour générer une prédiction cible.

9.  Procédé mis en œuvre par ordinateur selon l'une quelconque des revendications précédentes, dans lequel la génération des encodages de caractéristique multidimensionnels comprend, pour chaque variable de mesure physiologique, la réalisation d'un procédé de regroupement de données sur les valeurs concernées et la génération, en tant qu'encodages de caractéristique multidimensionnels, d'un vecteur de caractéristique pour chaque valeur.

10. Procédé mis en œuvre par ordinateur selon l'une quelconque des revendications précédentes, dans lequel la génération des corrélations spatiales comprend l'utilisation d'un mécanisme d'auto-attention clé-requête-valeur.

11. Procédé mis en œuvre par ordinateur selon l'une quelconque des revendications précédentes, dans lequel la génération des corrélations temporelles à court terme comprend l'utilisation d'un mécanisme basé sur l'attention clé-requête-valeur.

12. Procédé mis en œuvre par ordinateur selon l'une quelconque des revendications précédentes, dans lequel le processus de prédiction comprend la génération répétée desdites premières corrélations et la mise à jour des intégrations de nœud concernées, comprenant, pour chaque itération ultérieure, le fait de commencer avec les premières intégrations de nœud mises à jour de l'itération précédente au lieu des intégrations de nœud initiales.

13. Programme informatique qui, lorsqu'il est exécuté sur un ordinateur, amène l'ordinateur à effectuer un procédé comprenant :
    la réalisation d'un processus de prédiction, le processus de prédiction comprenant :

    sur la base de données d'entrée comprenant des valeurs de variables de mesure physiologiques d'un patient sur une période de temps, le calcul (S51) de premières corrélations dans les données d'entrée, le calcul comprenant le calcul de corrélations temporelles à court terme entre les valeurs de variables de mesure physiologiques à des étapes temporelles consécutives à l'aide d'un mécanisme basé sur l'attention et le calcul de corrélations spatiales entre les valeurs de différentes variables de mesure physiologiques à une même étape temporelle à l'aide d'un mécanisme d'auto-attention ;

la génération (S52) de premières intégrations de nœud mises à jour sur la base des données d'entrée et des premières corrélations, chaque nœud correspondant à une variable de mesure physiologique à une étape temporelle ;

à l'aide d'un réseau neuronal récurrent, RNN, la génération de corrélations temporelles à long terme entre les premières intégrations de nœud mises à jour et la mise à jour (S53) de la première intégration de nœud mise à jour sur la base des corrélations temporelles à long terme pour générer des intégrations mises à jour temporellement ; et

sur la base des intégrations mises à jour temporellement et à l'aide (S54) d'un réseau neuronal, NN, la génération d'une prédiction indiquant si le patient aura une septicémie.

14. Appareil de traitement d'informations (10) comprenant une mémoire (994) et un processeur (993) connecté à la mémoire (994), dans lequel le processeur (993) est configuré pour :
    réaliser un processus de prédiction, le processus de prédiction comprenant :

    sur la base de données d'entrée comprenant des valeurs de variables de mesure physiologiques d'un patient sur une période de temps, le calcul de premières corrélations dans les données d'entrée, comprenant le calcul de corrélations temporelles à court terme entre les valeurs de variables de mesure physiologiques à des étapes temporelles consécutives à l'aide d'un mécanisme basé sur l'attention et le calcul de corrélations spatiales entre les valeurs de différentes variables de mesure physiologiques à une même étape temporelle à l'aide d'un mécanisme d'auto-attention ;

    la génération de premières intégrations de nœud mises à jour sur la base des données d'entrée et des premières corrélations, chaque nœud correspondant à une variable de mesure physiologique à une étape temporelle ;

    à l'aide d'un réseau neuronal récurrent, RNN, la génération de corrélations temporelles à long terme entre les premières intégrations de nœud mises à jour et la mise à jour de la première intégration de nœud mise à jour sur la base des corrélations temporelles à long terme pour générer des intégrations mises à jour temporellement ; et

sur la base des intégrations mises à jour temporellement et à l'aide d'un réseau neuronal, NN, la génération d'une prédiction indiquant si le patient aura une septicémie.

20

Binary prediction

241

Temporal convolution
network

24

Temporal convolution
network block

e1    e2    e3

Weighted sum

Spatial cross-
correlation block
(Temporal graph
convolution network)

A: adjacency
matrix

23

A

Absolute
Absolute
Absolute
difference
between node
values (E_i - E_j)

E

High
dimensional
embedder

E

22

21

Input:
(x: traffic speed)
(S: Known
adjacency matrix)

Known
adjacency
matrix (S)

X

Input
block

FIG. 1 (related art)

FIG. 2 (related art)

30

FIG. 3 (related art)

FIG. 4

FIG. 5

First
Correlations
at time t

Temporal

Spatial

Spatial cross correlation at time t

Temp

O2Sat

Resp.
rate

Heart
rate

B.P.

Spatial dimension

Short range temportal cross-correlation
between time t-1 and t

Temporal dimension

Resp.
rate

Temp

O2Sat

Heart
rate

B.P.

Time step = t-1

Resp.
rate

Temp

O2Sat

Heart
rate

B.P.

Time step = t

Spatial with short-
range temporal
correlation at time t

$E_t$

43

Generate feature embeddings ($E'_t$)

431

$E'_t$

Generate short-term temporal
cross-correlation embedding at time t

$e_{t-1}$

432

$S_t$

Generate spatial cross-correlation
embedding at time t

433

$e_t$

FIG. 6

EP 4 517 775 B1

Raw input nodes: numerical values [1]

Dimension: [30x 6 x 1]
(time stamps x Number of features x numerical value) — $x$

Data imputation and pre-processing [2]

Dimension: [30x 6 x 1]
(time stamps x Number of features x numerical value) — $x$

High dimensional feature encoder [3]

Dimension: [30x 6 x 10]
(time stamps x Number of features x high dimensional feature representation) — $E$

Spatial with short-range temporal correlation block [4]

Dimension: [30x 6 x 10]
(time stamps x Number of features x high dimensional feature representation) — $e$

RNN-based network (GRU) [5]

Dimension: [30x 6 x 10]
(time stamps x Number of features x embedding vector) — $h$

Neural network [6]

Dimension: [10] — $Y_{t+T}$

Binary prediction [7]

Dimension: [30 x 6 x 10]
(time stamps x Number of features x high dimensional feature representation) — $E_t$

43

Generate feature embeddings ($E'_t$) [431]

$E'_t$ Dimension: [30x 6 x 10]

Generate short-term temporal cross-correlation embedding at time t [432]

$S_t$ Dimension: [30x 6 x 10]

Generate spatial cross-correlation embedding at time t [433]

$e_{t-1}$

$e_t$ Dimension: [30x 6 x 10]

EP 4 517 775 B1

FIG. 7

FIG. 8

EP 4 517 775 B1

| HR | O2Sat | Temp | SBP | MAP | DBP | Resp | EtCO2 | BaseExcess | HCO3 | ... | Fibrinogen | Platelets | Age | Gender | Unit1 | Unit2 |
|---|---|---|---|---|---|---|---|---|---|---|---|---|---|---|---|---|
| 0.0 | 0.0 | 0.00 | 0.0 | 0.00 | 0.0 | 0.0 | 0.0 | 0.0 | 0.0 | ... | 0.0 | 0.0 | 83.14 | 0 | 0.0 | 0.0 |
| 97.0 | 95.0 | 0.00 | 98.0 | 75.33 | 0.0 | 19.0 | 0.0 | 0.0 | 0.0 | ... | 0.0 | 0.0 | 83.14 | 0 | 0.0 | 0.0 |
| 89.0 | 99.0 | 0.00 | 122.0 | 86.00 | 0.0 | 22.0 | 0.0 | 0.0 | 0.0 | ... | 0.0 | 0.0 | 83.14 | 0 | 0.0 | 0.0 |
| 90.0 | 95.0 | 0.00 | 0.0 | 0.00 | 0.0 | 30.0 | 0.0 | 24.0 | 0.0 | ... | 0.0 | 0.0 | 83.14 | 0 | 0.0 | 0.0 |
| 103.0 | 88.5 | 0.00 | 122.0 | 91.33 | 0.0 | 24.5 | 0.0 | 0.0 | 0.0 | ... | 0.0 | 0.0 | 83.14 | 0 | 0.0 | 0.0 |
| 110.0 | 91.0 | 0.00 | 0.0 | 0.00 | 0.0 | 22.0 | 0.0 | 0.0 | 0.0 | ... | 0.0 | 0.0 | 83.14 | 0 | 0.0 | 0.0 |
| 108.0 | 92.0 | 36.11 | 123.0 | 77.00 | 0.0 | 29.0 | 0.0 | 0.0 | 0.0 | ... | 0.0 | 0.0 | 83.14 | 0 | 0.0 | 0.0 |
| 106.0 | 90.5 | 0.00 | 93.0 | 76.33 | 0.0 | 29.0 | 0.0 | 0.0 | 0.0 | ... | 0.0 | 0.0 | 83.14 | 0 | 0.0 | 0.0 |
| 104.0 | 95.0 | 0.00 | 133.0 | 88.33 | 0.0 | 26.0 | 0.0 | 0.0 | 0.0 | ... | 0.0 | 0.0 | 83.14 | 0 | 0.0 | 0.0 |
| 102.0 | 91.0 | 0.00 | 134.0 | 87.33 | 0.0 | 30.0 | 0.0 | 0.0 | 0.0 | ... | 0.0 | 0.0 | 83.14 | 0 | 0.0 | 0.0 |

FIG. 9

S51 — Compute first correlations

S52 — Generate first updated node embeddings

S53 — Update first updated node embeddings using RNN to generate temporally updated embeddings

S54 — Using a NN, generate a prediction (sepsis/no sepsis)

FIG. 10

**Training**

Start

S11 — Data preprocessing

S12 — Numerical data binning

S13 — Create windows of data

S14 — Load training windows data and labels

S15 — Generate node embeddings for each feature

S16 — Generate second updated node embeddings

S17 — Generate temporal embeddings

S18 — Classify it to sepsis/non-sepsis

S19 — Compute the loss for actual sepsis status at time t+T and predicted sepsis status at t+T

S20 — Is loss converged?

No

Yes

End

**Test**

Start

S31 — Data preprocessing

S32 — Numerical data binning

S33 — Create windows of data

S34 — Load test windows data and labels

S35 — Generate node embeddings for each feature

S36 — Generate second updated node embeddings

S37 — Generate temporal embeddings

S38 — Classify it to sepsis/non-sepsis

End

FIG. 11

Physiological measurement variables observation — A

Data imputation and data sampling — B

High dimensional encoding — C

Spatial with short-range temporal correlation block — D

Recurrent neural network (GRU) — E

Neural network — F

Sepsis / Non-sepsis classification — G

FIG. 12

10

| | | | |
|---|---|---|---|
| PROCESSOR | | MEMORY | |
| | 993 | | 994 |

992

| | | | | |
|---|---|---|---|---|
| | 995 | | 996 | |
| DISPLAY | | INPUT | | |
| | | | | 997 |
| | | | | NETWORK I/F |

FIG. 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JOSEPH FUTOMA et al.** Learning to Detect Sepsis with a Multitask Gaussian Process RNN Classifier. *arxiv.org, Cornell University Library, 201 Olin Library Cornell University Ithaca*, 13 June 2017